# EUROPÄISCHE PATENTSCHRIFT

(11) **EP 1 716 104 B1**
(45) Veröffentlichungstag und Bekanntmachung des Hinweises auf die Patenterteilung: **04.08.2010**
(21) Anmeldenummer: 05701203.1
(22) Anmeldetag: 27.01.2005
(51) Int. Cl.: C07C 253/10, C07C 253/34, C07C 255/07

(54) **VERFAHREN ZUR HERSTELLUNG VON 3-PENTENNITRIL**
METHOD FOR PRODUCING 3-PENTENENITRILE
PROCEDE POUR PRODUIRE DU 3-PENTENENITRILE

(30) Priorität: 29.01.2004 DE 102004004720
(43) Veröffentlichungstag der Anmeldung: 02.11.2006
(73) Patentinhaber: BASF SE, 67056 Ludwigshafen (DE)
(72) Erfinder: SCHEIDEL, Jens, 69493 Hirschberg (DE); JUNGKAMP, Tim, B-2950 Kapellen (BE); BARTSCH, Michael, 67433 Neustadt (DE); HADERLEIN, Gerd, 67269 Grünstadt (DE); BAUMANN, Robert, 68159 Mannheim (DE); LUYKEN, Hermann, 67069 Ludwigshafen (DE)
(86) Internationale Anmeldenummer: PCT/EP2005/000774
(87) Internationale Veröffentlichungsnummer: WO 2005/073171

(56) Entgegenhaltungen:
- DE-A1- 19 652 273
- R.KEESE ET AL.: "Fundamentals of Preparative Organic Chemistry" 1982, ELLIS HORWOOD LTD , XP002329986 Seiten 26-33

## Beschreibung

Die vorliegende Erfindung betrifft ein Verfahren zur Herstellung von 3-Pentennitril.

Adipodinitril ist ein wichtiges Ausgangsprodukt in der Nylonherstellung, das durch zweifache Hydrocyanierung von 1,3-Butadien erhalten wird. Dabei wird in einer ersten Hydrocyanierung 1,3-Butadien zu 3-Pentennitril hydrocyaniert, wobei als Nebenprodukte hauptsächlich 2-Methyl-3-butennitril, 4-Pentennitril, 2-Pentennitrile, 2-Methyl-2-butennitrile, C₉-Nitrile und Methylglutamitril erhalten werden. In einer zweiten, sich anschließenden Hydrocyanierung wird 3-Pentennitril mit Cyanwasserstoff zu Adipodinitril umgesetzt. Beide Hydrocyanierungen werden durch Nickel(0)-Phosphor-Komplexe katalysiert.

Für die zweite Hydrocyanierung ist es wesentlich, dass das eingesetzte 3-Pentennitril frei von 2-Methyl-3-butennitril ist, da anderenfalls 2-Methyl-3-butennitril zu dem unerwünschten Nebenprodukt Methylglutamitril hydrocyaniert würde.

Eine allgemeine Übersicht über die Nickel-katalysierte Olefinhydrocyanierung ist in Tolman et al., Adv. Cat. 33, 1-46 (1985) beschrieben.

Die Hydrocyanierung von 1,3-Butadien unter Verwendung eines Nickelkatalysators der Formel Ni[P(OR)₃]₄ wird in US 3,496,215 beschrieben. Nachteilig an diesem Verfahren ist, dass keine geeignete Technik zur vollständigen Rückgewinnung des 1,3-Butadiens oder des Katalysators angegeben ist.

US 5,693,843, US 5,696,280, US 5,821,378 und US 5,981,772 beschreiben Hydrocyanierungen von 1,3-Butadien mit multidentaten phosphorhaltigen Liganden, wobei jedoch in den einzelnen Ausführungsformen keine geeignete Verfahrensweise für die Rückgewinnung der Katalysatorkomponenten dargestellt sind.

Die Durchführung der Hydrocyanierung in einem oder mehreren Reaktoren und deren Verschaltung ist in US 4,810,815 beschrieben, wobei die Möglichkeit des kontinuierlichen Betriebs von Rührkesseln oder Kaskaden von Rührkesseln erwähnt wird, jedoch in Beispielen nur eine Semibatchfahnrveise im Detail beschrieben ist, woraus für den Fachmann nicht direkt abgeleitet werden kann, unter welchen Bedingungen die Fahrweise in kontinuierlichen Rührkesseln zu erfolgen hat.

D1 = DE-A-196 52 273 betrifft ein Verfahren zur Herstellung von Gemischen monoolefinischer Cs-Mononitrile mit nicht konjugierter C=C und CN-Bindung durch katalytische Hydrocyanierung eines butadien-haltigen Kohlenwasserstoffgemisches, indem man in dem Kohlenwasserstoffgemisch zunächst den Anteil desjenigen Komponenten, die die katalytische Hydrocyanierung beeinträchtigen, verringert und das so erhaltene Gemisch anschließend katalytisch hydrocyaniert.

Ein Verfahren zur Abtrennung von organischen phosphorhaltigen Verbindungen und ihrer Metallkomplexe von organischen Nitrilen in der Hydrocyanierung von Olefinen wird in US 3,773,809 beschrieben. Die Abtrennung erfolgt dabei durch Inkontaktbringen des Produktes mit einem Cycloparaffin oder paraffinartigen Kohlenwasserstoff. Dabei bildet sich ein flüssiges mehrphasiges System. Diese Methode der Abtrennung und Rückgewinnung von Katalysatorkomponenten durch Extraktion ist wegen der zu geringen Konzentration von Dinitrilen im Reaktionsprodukt bei der Hydrocyanierung von 1,3-Butadien nicht anwendbar.

Aufgabe der vorliegenden Erfindung ist es somit, ein integriertes Verfahren zur Herstellung von 3-Pentennitril durch Hydrocyanierung von 1,3-Butadien bereitzustellen, bei dem 3-Pentennitril im Wesentlichen frei von 2-Methyl-3-butennitril erhalten wird, das eingesetzte 1,3-Butadien zur Erhöhung der Verfahrensausbeute vorzugsweise zurückgeführt wird und der Katalysator zum Zwecke seines wirtschaftlichen Einsatzes von den Pentennitrilen vorzugsweise abgetrennt und zurückgeführt wird.

Es ist bekannt, dass 2-Methyl-3-butennitril unter den Hydrocyanierungsbedingungen, insbesondere in Gegenwart von Nickel(0)-Komplexen zu Methylglutardinitril reagiert. Daher ist es eine weitere Aufgabe der vorliegenden Erfindung, ein Verfahren zur Herstellung von 3-Pentennitril durch Hydrocyanierung von 1,3-Butadien bereitzustellen, bei dem vorzugsweise möglichst wenig 2-Methyl-3-butennitril in die Hydrocyanierung zurückgefahren wird. Daher sollten in dem erfindungsgemäßen Verfahren der rückgeführte Katalysatorstrom und der rückgeführte Teil des 1,3-Butadiens möglichst weitgehend von 2-Methyl-3-butennithl befreit werden.

Des Weiteren sind homogen gelöste Hydrocyanierungskatalysatoren bekanntermaßen thermisch labil. Deshalb ist es eine weitere Aufgabe der vorliegenden Erfindung, ein Verfahren zur Herstellung von 3-Pentennitril durch Hydrocyanierung von 1,3-Butadien bereit zu stellen, bei dem der Katalysator vorzugsweise einer möglichst niedrigen thermischen Belastung ausgesetzt wird.

Erfindungsgemäß wird diese Aufgabe gelöst durch ein Verfahren zur Herstellung von 3-Pentennitril.

Das erfindungsgemäße Verfahren ist durch die folgenden Verfahrensschritte gekennzeichnet:
(a) Umsetzung von 1,3-Butadien mit Cyanwasserstoff an mindestens einem Katalysator unter Erhalt eines Stromes 1, der 3-Pentennitril, 2-Methyl-3-butennitril, den mindestens einen homogen gelösten Nickel(0)-Katalysator, der mit Phosphorliganden stabilisiert ist und die Phosphorliganden ausgewählt sind aus der Gruppe, bestehend aus Phosphinen, Phosphiten, Phosphiniten und Phosphoniten und 1,3-Butadien enthält,
(b) Destillation des Stromes 1 in einer Kolonne unter Erhalt eines an 1,3-Butadien reichen Stromes 2 als Kopfprodukt und eines an 1,3-Butadien armen Stromes 3 als Sumpfprodukt, der 3-Pentennitril, den mindestens einen Katalysator und 2-Methyl-3-butennitril enthält,
(c) Destillation des Stromes 3 in einer Kolonne unter Erhalt eines Stromes 4 als Kopfprodukt, der 1,3-Butadien enthält, eines Stromes 5 an einem Seitenabzug der Kolonne, der 3-Pentennitril und 2-Methyl-3-butennitril enthält, und eines Stromes 6 als Sumpfprodukt, der den mindestens einen Katalysator enthält,
(d) Destillation des Stromes 5 unter Erhalt eines Stromes 7 als Kopfprodukt, der 2-Methyl-3-butennitril enthält, und eines Stromes 8 als Sumpfprodukt, der 3-Pentennitril enthält,
   mit der Maßgabe, dass in den Verfahrensschritten (b) und (c) die Sumpftemperaturen 140°C nicht übersteigen und die Summe der mittleren Verweilzeiten in den Desbilationsvorrichtungen in den Verfahrensschritten (b) und (c) zusammen nicht größer als 10 Stunden ist.

Der ***Verfahrensschritt (a)*** umfasst die Umsetzung von 1,3-Butadien und Cyanwasserstoff an mindestens einem Katalysator. Als Katalysator werden Nickel(0)-Katalysatoren-Komplexe verwendet.

Bei den Ni(0)-Komplexen, die phosphorhaltige Liganden und/oder freie phosphorhaltige Liganden enthalten, handelt es sich bevorzugt um homogen gelöste Nickei(0)-Komplexe.

Die phosphorhaltigen Liganden der Nickei(0)-Komplexe und die freien phosphorhaltigen Liganden sind vorzugsweise ausgewählt aus mono- oder bidentaten Phosphinen, Phosphiten, Phosphiniten und Phosphoniten.

Diese phosphorhaltigen Liganden weisen vorzugsweise die Formel I auf:

P(X¹R¹)(X²R²)(X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen I können solche der Formel I a

(o-Tolyl-O-)_{w} (m-Tolyl-O-)ₓ (p-Tolyl-O-)_{y} (Phenyl-O-)_{z} P (I a)

eingesetzt werden, wobei w, x, y und z eine natürliche Zahl bedeuten, und folgende Bedingungen gelten: w + x + y + z = 3 und w, z ≤ 2.

Solche Verbindungen I a sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P, (m-Tolyl-O-)₂(Phertyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tolyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P(O-R¹)ₓ (O-R²)_{y} (O-R³)_{z} (O-R⁴)ₚ (I b)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromati- schen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoff- atom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der a- romatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet,
- R⁴:: oder mit einem aromatischen Substituenten in p-Stellung zu dem Sauerstoffatom, das das Phosphoratom mit dem aromatischen System verbindet, wobei der aro- matische Rest in o-Stellung zu dem Sauerstoffatom, das das Phosphoratom mit dem aromatischen System verbindet, ein Wasserstoffatom trägt, aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x :: 1 oder 2,
- y, z, p:: unabhängig voneinander 0, 1 oder 2 mit der Maßgabe, dass x+y+z+p = 3.

Bevorzugte Phosphite der Formel I b sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest R² sind m-Tolyl-; m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung I b ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel I b sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel I b sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel I b können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite I b und zur Aufarbeitung sind der DE-A 199 53 058 zu entnehmen.

Die Phosphite I b können auch in Form eines Gemisches verschiedener Phosphite I b als Ligand verwendet werden. Ein solches Gemisch kann beispielsweise bei der Herstellung der Phosphite I b anfallen.

Es ist allerdings bevorzugt, dass der phosphorhaltige Ligand mehrzähnig, insbesondere zweizähnig ist. Daher weist der verwendete Ligand vorzugsweise die Formel II 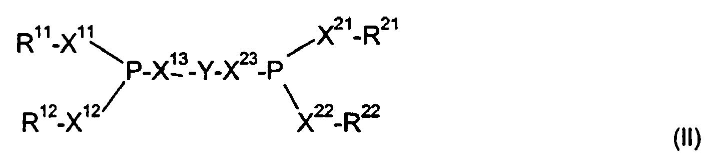 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- Y: Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder uriterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, I a, I b und II sowie deren Herstellung sind an sich bekannt. Als phosphorhaltiger Ligand können auch Mischungen, enthaltend mindestens zwei der Verbindungen I, I a, I b und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel I b

P(O-R¹)ₓ (O-R²)_{y} (O-R³)_{z} (O-R⁴)ₚ (I b)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen.

Der Verfahrensschritt (a) des erfindungsgemäßen Verfahrens kann in jeder geeigneten, dem Fachmann bekannten Vorrichtung durchgeführt werden. Für die Reaktion kommen somit übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei, Apparaturen durchgeführt werden.

In einer bevorzugten Ausführungsform des erfindungsgemäßen Verfahrens haben sich Reaktoren mit Rückvermischuhgscharakteristik oder Kaskaden von Reaktoren mit Rückvermischungscharakteristik als vorteilhaft erwiesen. Als besonders vorteilhaft haben sich Kaskaden aus Reaktoren mit Rückvermischungscharakteristik erwiesen, die in Bezug auf die Dosierung von Cyanwasserstoff in Querstromfahrweise betrieben werden.

Die Hydrocyanierung kann in Gegenwart oder in Abwesenheit von einem Lösemittel durchgeführt werden. Wenn ein Lösemittel verwendet wird, so sollte das Lösemittel bei der gegebenen Reaktionstemperatur und dem gegebenen Reaktionsdruck flüssig und inert gegenüber den ungesättigten Verbindungen und dem mindestens einen Katalysator sein. Im Allgemeinen werden als Lösemittel Kohlenwasserstoffe, beispielsweise Benzol oder Xylol, oder Nitrile, beispielsweise Acetonitril oder Benzonitril, verwendet. Vorzugsweise wird allerdings ein Ligand als Lösemittel verwendet.

Die Reaktion kann in Batchfahrweise, kontinuierlich oder im Semibatchbetrieb durchgeführt werden.

Die Hydrocyanierungsreaktion kann durchgeführt werden, in dem die Vorrichtung mit allen Reaktanten bestückt wird. Bevorzugt ist allerdings, wenn die Vorrichtung mit dem Katalysator, der ungesättigten organischen Verbindung und gegebenenfalls dem Lösemittel gefüllt wird. Vorzugsweise schwebt der gasförmige Cyanwasserstoff über der Oberfläche der Reaktionsmischung oder wird durch die Reaktionsmischung geleitet. Eine weitere Verfahrensweise zum Bestücken der Vorrichtung ist das Befüllen der Vorrichtung mit dem Katalysator, Cyanwasserstoff und gegebenenfalls dem Lösemittel und das langsame Zuspeisen der ungesättigten Verbindung zu der Reaktionsmischung. Alternativ ist auch möglich, dass die Reaktanten in den Reaktor eingeführt werden und die Reaktionsmischung auf die Reaktionstemperatur gebracht wird, bei welcher der Cyanwasserstoff flüssig zu der Mischung gegeben wird. Darüber hinaus kann der Cyanwasserstoff auch vor dem Erwärmen auf Reaktionstemperatur zugegeben werden. Die Reaktion wird unter konventionellen Hydrocyanierungsbedingungen für Temperatur, Atmosphäre, Reaktionszeit, etc. durchgeführt.

Vorzugsweise wird die Hydrocyanierung kontinuierlich in einem oder mehreren gerührten Verfahrensschritten durchgeführt. Wenn eine Mehrzahl von Verfahrensschritten verwendet wird, so ist es bevorzugt, dass die Verfahrensschritte in Serie geschaltet sind. Dabei wird das Produkt von einem Verfahrensschritt direkt in den nächsten Verfahrensschritt überführt. Der Cyanwasserstoff kann direkt in den ersten Verfahrensschritt oder zwischen den einzelnen Verfahrensschritten zugeführt werden.

Wenn das erfindungsgemäße Verfahren im Semibatchbetrieb durchgeführt wird, so ist es bevorzugt, dass im Reaktor die Katalysatorkomponenten und 1,3-Butadien vorgelegt werden, während Cyanwasserstoff über die Reaktionszeit hinweg in die Reaktionsmischung dosiert wird.

Die Reaktion wird vorzugsweise bei absoluten Drücken von 0,1 bis 500 MPa, besonders bevorzugt 0,5 bis 50 MPa, insbesondere 1 bis 5 MPa, durchgeführt. Die Reaktion wird vorzugsweise bei Temperaturen von 273 bis 473 K, besonders bevorzugt 313 bis 423 K, insbesondere bei 333 bis 393 K, durchgeführt. Dabei haben sich durchschnittliche mittlere Verweilzeiten der flüssigen Reaktorphase im Bereich von 0,001 bis 100 Stunden, vorzugsweise 0,05 bis 20 Stunden, besonders bevorzugt 0,1 bis 5 Stunden, jeweils pro Reaktor, als vorteilhaft erwiesen.

Die Reaktion kann in einer Ausführungsform in flüssiger Phase in Gegenwart einer Gasphase und gegebenenfalls einer festen suspendierten Phase ausgeführt werden. Dabei können die Ausgangsstoffe Cyanwasserstoff und 1,3-Butadien jeweils flüssig oder gasförmig zudosiert werden.

Die Reaktion kann in einer weiteren Ausführungsform in flüssiger Phase durchgeführt werden, wobei der Druck im Reaktor so bemessen ist, dass alle Einsatzstoffe wie 1,3-Butadien, Cyanwasserstoff und der mindestens eine Katalysator flüssig zudosiert werden und in der Reaktionsmischung in flüssiger Phase vorliegen. Dabei kann eine feste suspendierte Phase im Reaktionsgemisch vorliegen, die auch zusammen mit dem mindestens einen Katalysator zudosiert werden kann, beispielsweise bestehend aus Abbauprodukten des Katalysatorsystems, enthaltend unter anderem Nickel(II)-Verbindungen.

Im Verfahrensschritt (a) wird ein Strom 1, der 3-Pentennitril, 2-Methyl-3-butennitril, den mindestens einen Katalysator und nicht umgesetztes 1,3-Butadien sowie Reste von nicht umgesetztem Cyanwasserstoff enthält, erhalten. Dieser Strom 1 weist vorzugsweise die folgende Zusammensetzung auf: 1 bis 80 Gew.-%, besonders bevorzugt 5 bis 50 Gew.-%, des mindestens einen Katalysators, 0,1 bis 50 Gew.-%, besonders bevorzugt 1 bis 25 Gew.-%, 1,3-Butadien, 1 bis 80 Gew.-%, besonders bevorzugt 10 bis 50 Gew.-%, Pentennitrile, umfassend trans-3-Pentennitril, 2-Methyl-3-butennitril sowie weitere Pentennitrilisomere und 0,1 Gew.-ppm bis 10 Gew.-%, besonders bevorzugt 10 Gew.-ppm bis 1 Gew.-%, Cyanwasserstoff jeweils bezogen auf die Gesamtmasse des Stromes 1.

Der Strom 1, der 3-Pentennitril, 2-Methyl-3-butennitril, den mindestens einen Katalysator und nicht umgesetztes 1,3-Butadien enthält, wird anschließend in ***Verfahrensschritt (b)*** in eine Destillationsvorrichtung überführt. In dieser Destillationsvorrichtung erfolgt eine Destillation des Stromes 1 unter Erhalt eines an 1,3-Butadien reichen Stromes 2 als Kopfprodukt und eines an 1,3-Butadien armen Stromes 3 als Sumpfprodukt, der 3-Pentennitril, den mindestens einen Katalysator und 2-Methyl-3-butennitril enthält.

Der Verfahrensschritt (b) des erfindungsgemäßen Verfahrens kann in in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer einzigen Apparatur durchgeführt werden.

In einer bevorzugten Ausführungsform des erfindungsgemäßen Verfahrens sind in der Destillationsvorrichtung Kolonneneinbauten mit strukturierter Packung vorhanden, die vorzugweise zwischen 2 und 60, besonders bevorzugt zwischen 3 und 40, insbesondere zwischen 4 und 20, Trennstufen erzeugen.

In einer besonders bevorzugten Ausführungsform des erfindungsgemäßen Verfahrens wird die mindestens eine zur Destillationsvorrichtung von Verfahrensschritt (b) gehörige Verdampferstufe so ausgeführt, dass das zu verdampfende Material möglichst wenig thermische Schädigung erleidet, wie es beispielsweise durch Fallfilmverdampfer, Mehrphasenwendelrohrverdampfer, Dünnschichtverdampfer oder Kurzwegverdampfer durch kurze Kontaktzeiten des Materials an der Verdampferoberfläche und möglichst geringe Temperaturen der Verdampferoberflächen erreicht wird.

In einer bevorzugten Ausführungsform des erfindungsgemäßen Verfahrens wird die Destillationsvorrichtung von Verfahrensschritt (b) mit einem geteilten Sumpf betrieben, wobei man aus einem ersten Sumpf der betreffenden Destillationskolonne einen im Verhältnis zum Strom 3 im Allgemeinen um ein Vielfaches größeren Umlaufstrom zum Verdampfer fährt, den flüssigen Ablaufstrom aus dem Verdampfer jedoch nicht direkt in den ersten Sumpf zurückgibt, sondern in einen zweiten Sumpf, der vom ersten Sumpf getrennt ist, auffängt, aus dem zweiten Sumpf den Strom 3 erhält und den verbleibenden Überschuss vom Verdampferumlaufstrom in den ersten Sumpf überlaufen lässt, wobei als Strom 3 aus dem zweiten Sumpf eine Mischung erhalten wird, die gegenüber dem aus dem ersten Sumpf abgezogenen Verdampferumlaufstrom an Leichtsiedern abgereichert ist. Als Verdampfer wird dabei vorzugsweise ein Fallfilmverdampfer verwendet.

In einer weiteren bevorzugten Ausführungsform des erfindungsgemäßen Verfahrens wird die Destillation bei mittleren Verweilzeiten der flüssigen Phase im Sumpfbereich der ein oder mehreren Destillationsapparaturen von zusammen weniger als 10 Stunden, besonders bevorzugt weniger als 5 Stunden, insbesondere weniger als 1 Stunde, durchgeführt.

In einer weiteren bevorzugten Ausführungsform des erfindungsgemäßen Verfahrens wird die Kondensation am Kopf der Destillationsvorrichtung so durchgeführt, dass ein Teilstrom vom Kopfaustrag in den Kondensator zurückgespült wird.

Die Destillation kann in einer weiteren bevorzugten Ausführungsform des erfindungsgemäßen Verfahrens mit einem Direktkondensator ausgeführt werden, so dass die Kondensation in einem Kolonnenschuss durchgeführt wird, der vorzugsweise ausgestattet ist mit einer strukturierten Kolonnenpackung, einer Fangtasse unterhalb dieser Packung, einem flüssigen Abzug aus der Fangtasse, einem an den flüssigen Abzug angeschlossenen Umpumpkreislauf mit Pumpe und Wärmetauscher sowie mindestens einer Vorrichtung zur Aufgabe des umgepumpten Flüssigstroms auf die Packung oberhalb der Fangtasse.

Um eine möglichst hohe Verfahrensausbeute bezüglich 1,3-Butadien trotz der nur teilweise erfolgten Umsetzung in Schritt (a) zu erreichen, ist es bevorzugt, dass der an 1,3-Butadien reiche Strom 2 in den Verfahrensschritt (a) zurückgeführt wird. Die Rückführung des Stromes 2 in Verfahrensschritt (a) kann gegebenenfalls auch nur teilweise erfolgen.

In einer weiteren Ausführungsform kann bei der Destillation des Schrittes (b) das für die Umsetzung in Verfahrensschritt (a) zusätzlich benötigte 1,3-Butadien in den Kopfbereich der Kolonne oder in den Strom 2 zugefügt werden.

In einer weiteren Ausführungsform enthält das zugefügte 1,3-Butadien einen Stabilisator, wie tert.-Butylbrenzkatechin oder 2,6-Di-tert.-butyl-para-kresol, gemäß Beschreibung in "Ullmann's Encyclopedia Of Industrial Chemistry, 6th Edition, 2000 Electronic Release, Kapitel "Butadiene - 6. Stabilization, Storage and Transportation".

In einer besonders bevorzugten Ausführungsform des erfindungsgemäßen Verfahrens wird das entweder direkt im Verfahrensschritt (a) eingesetzte oder zu Schritt (b) zugefügte und über Strom 2 in Schritt (a) überführte 1,3-Butadien durch Kontaktieren mit Molsieb mit einer Porengröße kleiner 10 Ångström oder durch Kontaktieren mit Aluminiumoxid von Wasser und gegebenenfalls dem Stabilisator befreit.

In einer weiteren besonders bevorzugten Ausführungsform wird das direkt in Verfahrensschritt (a) eingesetzte oder das in den Strom 2 zugeführte 1,3-Butadien ohne Stabilisator eingesetzt, wobei durch eine geeignete Wahl der Druckverhältnisse die Kondensationstemperaturen im Kopfbereich der Destillationseinrichtung von Verfahrensschritt (b) kleiner 293 K gehalten werden, um eine Polymerisation von 1,3-Butadien zu verhindern, insbesondere um das Wachstum von Popcorn-Polymerkeimen zu begrenzen.

Der absolute Druck in Verfahrensschritt (b) beträgt vorzugsweise 0,001 bis 100 bar, besonders bevorzugt 0,01 bis 10 bar, insbesondere 0,5 bis 5 bar. Die Destillation wird so durchgeführt, dass die Temperatur im Sumpf der Destillationsvorrichtung vorzugsweise 30 bis 140 °C, besonders bevorzugt 50 bis 130 °C, insbesondere 60 bis 120 °C, beträgt. Die Destillation wird so durchgeführt, dass die Kondensationstemperatur am Kopf der Destillationsvorrichtung vorzugsweise -50 bis 140 °C, besonders bevorzugt - 15 bis 60 °C, insbesondere 5 bis 45 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

Das Rücklaufverhältnis am Kopf der Destillationsvorrichtung wird vorzugsweise so eingestellt, dass der Strom 2 1 bis 1000 ppm, besonders bevorzugt 5 bis 500 ppm, insbesondere 10 bis 200 ppm, 2 Methyl-3-butennitril enthält.

Dies trägt dazu bei, dass das rückgeführte 1,3-Butadien wenig 2-Methyl-3-butennitril enthält, das in Verfahrensschritt (a) zu Methylglutardinitril reagiert.

In Verfahrensschritt (b) wird ein an 1,3-Butadien reicher Strom 2 als Kopfprodukt und ein an 1,3-Butadien armer Strom 3 als Sumpfprodukt erhalten. Die Bezeichnung der Ströme als an 1,3-Butadien reich bzw. arm bezieht sich dabei auf den Gehalt an 1,3-Butadien des in Verfahrensschritt (b) eingesetzten Stromes 1.

In einer bevorzugten Ausführungsform des erfindungsgemäßen Verfahrens enthält der an 1,3-Butadien reiche Strom 2 in Summe 50 bis 100 Gew.-%, besonders bevorzugt 80 bis 100 Gew.-%, insbesondere 85 bis 99 Gew.-%, 1,3-Butadien und Buten-Isomere sowie in Summe 0 bis 50 Gew.-%, besonders bevorzugt 0 bis 20 Gew.-%, insbesondere 10 Gew.-ppm bis 1 Gew.-%, Pentennitril-Isomere, von denen im Wesentlichen 2-Methyl-3-butennitril und trans-3-Pentennitril im Strom 2 vertreten sind.

In einer bevorzugten Ausführungsform des erfindungsgemäßen Verfahrens enthält der an 1,3-Butadien arme Strom 3 in Summe 0 bis 50 Gew.-%, besonders bevorzugt 1 bis 30 Gew.-%, insbesondere 2 bis 20 Gew.-%, 1,3-Butadien und Buten-Isomere, jeweils bezogen auf die Gesamtmasse des Stromes 3. In einer besonders bevorzugten Ausführungsform des erfindungsgemäßen Verfahrens werden die zuvor genannten Spezifikationen an 1,3-Butadien sowohl im Strom 2 als auch im Strom 3 erreicht.

Der aus Verfahrensschritt (b) stammende an 1,3-Butadien arme Strom 3, der 3-Pentennitril, den mindestens einen Katalysator und 2-Methyl-3-butennitril enthält, wird anschließend in ***Verfahrensschritt (c)*** in eine Destillationsvorrichtung überführt. In dieser Destillationsvorrichtung erfolgt eine Destillation des Stromes 3 unter Erhalt eines Stromes 4 als Kopfprodukt, der 1,3-Butadien enthält, eines Stromes 5 an einem Seitenabzug der Kolonne, der 3-Pentennitril und 2-Methyl-3-butennitril enthält, und eines Stromes 6 als Sumpfprodukt, der den mindestens einen Katalysator enthält.

Der Verfahrensschritt (c) des erfindungsgemäßen Verfahrens kann in jeder geeigneten, dem Fachmann bekannten Vorrichtung durchgeführt werden. Für diese Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer Apparatur durchgeführt werden.

In einer besonders bevorzugten Ausführungsform umfasst die Destillationsvorrichtung in Verfahrensschritt (c) mindestens eine Destillationskolonne mit einem Abtriebsteil. Insbesondere bevorzugt ist eine Ausführungsform, die als Destillationsvorrichtung in Verfahrensschritt (c) nur eine Destillationskolonne enthält, die in Abtriebsfahrweise betrieben wird.

Die Destillationskolonne in der Destillationsvorrichtung ist vorzugsweise mit einer strukturierten Packung ausgestattet, die 2 bis 50, besonders bevorzugt 3 bis 40, insbesondere 4 bis 30, theoretische Trennstufen erzeugt.

In einer besonders bevorzugten Ausführungsform des erfindungsgemäßen Verfahrens werden die mindestens eine zur Destillationsvorrichtung von Verfahrensschritt (c) gehörigen Verdampferstufen so ausgeführt, dass das zu verdampfende Material möglichst wenig thermische Schädigung erleidet, wie es beispielsweise durch Fallfilmverdampfer, Mehrphasenwendelrohrverdampfer, Dünnschichtverdampfer oder Kurzwegverdampfer, durch kurze Kontaktzeiten des Materials an der Verdampferoberfläche und möglichst geringe Temperaturen der Verdampferoberflächen erreicht wird.

Der absolute Druck in Verfahrensschritt (c) beträgt vorzugsweise 0,001 bis 10 bar, besonders bevorzugt 0,010 bis 1 bar, insbesondere 0,02 bis 0,5 bar. Die Destillation wird so durchgeführt, dass die Temperatur im Sumpf der Destillationsvorrichtung vorzugsweise 30 bis 140 °C, besonders bevorzugt 50 bis 130 °C, insbesondere 60 bis 120 °C, beträgt. Die Destillation wird so durchgeführt, dass die Kondensationstemperatur am Kopf der Destillationsvorrichtung vorzugsweise -50 bis 140 °C, besonders bevorzugt - 15 bis 60 °C, insbesondere 5 bis 45 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

In einer weiteren bevorzugten Ausführungsform des erfindungsgemäßen Verfahrens werden somit in den Verfahrensschritten (b) und (c) eine Sumpftemperatur von 140 °C nicht überstiegen.

In einer weiteren bevorzugten Ausführungsform des erfindungsgemäßen Verfahrens wird die Destillation bei mittleren Verweilzeiten der flüssigen Phase im Sumpfbereich der ein oder mehreren Destillationsapparaturen von zusammen weniger als 10 Stunden, besonders bevorzugt weniger als 5 Stunden, insbesondere weniger als 1 Stunde, durchgeführt.

In einer besonders bevorzugten Ausführungsform des erfindungsgemäßen Verfahrens wird die Destillation bei mittleren Verweilzeiten der flüssigen Phase im Sumpfbereich aller Destillationsapparaturen in den Verfahrensschritten (b) und (c) von zusammen weniger als 10 Stunden, besonders bevorzugt weniger als 5 Stunden, insbesondere weniger als 1 Stunde, durchgeführt.

In der Destillation des Verfahrensschrittes (c) wird ein Strom 4 als Kopfprodukt erhalten. Dieser Strom 4 enthält vorzugsweise in Summe 50 bis 100 Gew.-%, besonders bevorzugt 80 bis 100 Gew.-%, insbesondere 90 bis 99,9 Gew.-%, 1,3-Butadien und Buten-Isomere sowie in Summe 0 bis 50 Gew.-%, besonders bevorzugt 0 bis 20 Gew.-%, insbesondere 10 Gew.-ppm bis 10 Gew.-%, Pentennitril-Isomere, von denen im Wesentlichen 2-Methyl-3-butennitril und trans-3-Pentennitril im Strom 4 vertreten sind.

In einer bevorzugten Ausführungsform des erfindungsgemäßen Verfahrens wird der Strom 4 gasförmig in mindestens einem Kondensator am Kopf der Destillationsvorrichtung erhalten, wobei in dem mindestens einen Kondensator Pentennitril-Komponenten aus dem Brüdenstrom der Destillationsvorrichtung von Verfahrensschritt (c) im oben genannten Bereich von Kondensationsbedingungen wie Druck und Temperatur zumindest teilweise auskondensiert werden und in die Kolonne zumindest teilweise flüssig als Pentennitrile sowie 1,3-Butadien und Buten-Isomere enthaltender Strom zurückgeführt werden.

Um die Verfahrensausbeute an eingesetztem 1,3-Butadien in dem erfindungsgemäßen Verfahren zu erhöhen, ist es bevorzugt, dass der Strom 4 in den Verfahrensschritt (a) zurückgeführt wird. Die Rückführung des Stromes 4 in Verfahrensschritt (a) kann gegebenenfalls auch nur teilweise erfolgen. Dabei kann der Strom 4 vor seiner Rückführung zusätzlich einer verfahrenstechnischen Aufarbeitung, beispielsweise einer Verdichtung auf einen höheren Druck, unterzogen werden.

In einer besonders bevorzugten Ausführungsform des erfindungsgemäßen Verfahrens wird der Strom 4 über den Verfahrensschritt (b) in den Verfahrensschritt (a) zurückgeführt, wobei die Pentennitril-Komponenten, die je nach Destillationsbedingungen in Strom 4 enthalten sein können, durch Zurückführen des Stromes 4 in die Destillationsvorrichtung von Verfahrensschritt (b) vorzugsweise aus dem Strom 4 abgetrennt werden und letztendlich nur der 1,3-Butadien- und Buten-Isomeren-Anteil von Strom 4 über Strom 2 in Schritt (a) zurückgeführt wird. In dem Verfahrensschritt (c) wird neben dem Strom 4 ein weiterer Strom 5 erhalten, der an einem Seitenabzug der Kolonne gewonnen wird. Dieser Strom 5 enthält 3-Pentennitril und 2-Methyl-3-butennitril neben anderen Pentennitril-Isomeren und Restbestandteilen an 1,3-Butadien und Buten-Isomeren. Der Anteil an 3-Pentennitril und 2-Methyl-3-butennitril in dem Strom 5 beträgt in Summe vorzugsweise 80 bis 100 Gew.-%, besonders bevorzugt 85 bis 99,998 Gew.-%, insbesondere 90 bis 99,9 Gew.-%, jeweils bezogen auf den Strom 5. Der Anteil an 1,3-Butadien und Buten-Isomeren in dem Strom 5 beträgt vorzugsweise 0 bis 20 Gew.-%, besonders bevorzugt 10 Gew.-ppm bis 5 Gew.-%, insbesondere 50 Gew.-ppm bis 2 Gew.-%, jeweils bezogen auf den Strom 5. Der Strom 5 wird vorzugsweise dampfförmig entnommen.

Der Seitenabzug der Destillationsvorrichtung befindet sich vorzugsweise unterhalb der Zulaufstelle von Strom 3, besonders bevorzugt in einer Position entsprechend 1 bis 20, insbesondere 2 bis 10, destillativen Trennstufen unterhalb der Zulaufstelle von Strom 3.

Als Sumpfprodukt erhält man im Verfahrensschritt (c) einen Strom 6, der den mindestens einen Katalysator sowie trans-3-Pentennitril und 2-Methyl-3-butennitril enthält. Der Anteil an Pentennitril-Isomeren in dem Strom 6 beträgt in Summe vorzugsweise 0,1 bis 80 Gew.-%, besonders bevorzugt 5 bis 50 Gew.-%, insbesondere 10 bis 40 Gew.-%, jeweils bezogen auf den Strom 6.

Dabei ist es besonders bevorzugt, dass der Strom 6 in den Verfahrensschritt (a) der Hydrocyanierung zumindest teilweise zurückgeführt wird, wobei eine Regeneration, wie in der DE-A-103 51 002 beschrieben ist. In einer weiteren Ausführungsform des erfindungsgemäßen Verfahrens kann die Destillationseinrichtung von Verfahrensschritt (c) mit einem oder mehreren weiteren flüssigen oder dampfförmigen Seitenabzügen, oberhalb oder unterhalb der Zulaufstelle von Strom 3, betrieben werden, um Ausschleus- oder Rückführströme zu entnehmen.

Zudem ist es auch möglich, den Strom 6 aus Verfahrensschritt (c) ganz oder teilweise als Katalysatorstrom für andere Hydrocyanierungen zu verwenden, beispielsweise zur Hydrocyanierung von 3-Pentennitril. Wenn der Katalysatorstrom 6 zur Hydrocyanierung von 3-Pentennitril verwendet wird, ist es bevorzugt, dass der Gehalt an 2-Methyl-3-butennitril in diesem Katalysatorstrom 6 möglichst gering ist.

Daher wird in einer bevorzugten Ausführungsform des erfindungsgemäßen Verfahrens in Verfahrensschritt (c) die Position des Seitenabzugs und die Gesamtzahl der theoretischen Trennstufen der Destillationsvorrichtung in Verfahrensschritt (c) so gewählt, dass über Sumpf der Strom 6 mit einer Konzentration von 2-Methyl-3-butennitril gewonnen wird, die im Vergleich zum Strom 5 erniedrigt ist, wobei sich die Erniedrigung auf das Verhältnis der Konzentrationen von 2-Methyl-3-butennitril zu trans-3-Pentennitril bezieht. Besonders bevorzugt sind 1 bis 50, insbesondere 2 bis 20, destillative Trennstufen zwischen der Position des Seitenabzugs und dem Sumpf. Diese Abreicherung von 2-Methyl-3-butennitril kann gegebenenfalls auch in einer separaten Vorrichtung, ausgeführt als Destillationskolonne mit einem Abtriebsteil, erfolgen. Vorzugsweise beträgt der Anteil an 2-Methyl-3-butennitril in dem Katalysatorstrom 6 0 bis 5 Gew.-%, besonders bevorzugt 10 Gew.-ppm bis 2 Gew.-%, insbesondere 50 Gew.-ppm bis 0,5 Gew.-%, bezogen auf den Katalysatorstrom 6. In einer weiteren Ausführungsform des erfindungsgemäßen Verfahrens kann der Strom 6 nach Abnahme eines Teilstromes 6b zu Zwecken der Ausschleusung, Regenerierung oder Verwendung in einem anderen Hydrocyanierungsverfahren, beispielsweise von 3-Pentenntril zu Adipodinitril, durch einen Strom an Frischkatalysator ergänzt werden, um die nötige Menge des mindestens einen Katalysators in Verfahrensschritt (a) sicherzustellen. Der Frischkatalysatorstrom kann aus einer gezielten Synthese, aus einem Regenerierverfahren oder aus einem Verfahren zur Rückgewinnung des Katalysators aus einem Hydrocyanierungsverfahren stammen, insbesondere aus einem Extraktionsverfahrensschritt in einem Verfahren zur Hydrocyanierung von 3-Pentennitril zu Adipodinitril.

In einer bevorzugten Ausführungsform wird der Frischkatalysatorstrom entweder direkt dem Verfahrensschritt (a) beziehungsweise zu Strom 6 nach der Stelle zugeführt, wo der Teilstrom 6b entnommen wurde.

In einer weiteren bevorzugten Ausführungsform wird der Frischkatalysatorstrom in die Destillationsvorrichtung von Verfahrensschritt (c) gefahren, um den Pentennitril-Gehalt des gesamten Katalysatorstroms zu Verfahrensschritt (a) in den oben angegebenen Grenzen kontrollieren zu können.

In einer weiteren bevorzugten Ausführungsform des erfindungsgemäßen Verfahrens wird die Menge der Katalysatorausschleusung und damit die nötige Ergänzungsmenge an Frischkatalysator so bemessen, dass im Katalysatorkreislauf der Gehalt an Methlyglutamitril nicht über 50 Gew.-%, besonders bevorzugt nicht über 20 Gew.-%, insbesondere nicht über 10 Gew.-%, jeweils bezogen auf den Katalysatorkreislauf, steigt, um den jeweils ausgeschleusten Katalysatorstrom in einer Regenerierung mit möglichst wenig hemmenden Effekten von Methylglutamitril zur Aufnahme von Nickel(0) vorliegen zu haben.

In einer weiteren bevorzugten Ausführungsform des erfindungsgemäßen Verfahrens wird die Menge der Katalysatorausschleusung und damit die nötige Ergänzungsmenge an Frischkatalysator so bemessen, dass im Katalysatorkreislauf der Gehalt an Nickel(0)-Komplexen nicht unter 0,05 Gew.-% fällt, besonders bevorzugt nicht unter 0,1 Gew.-%, insbesondere nicht unter 0,2 Gew.-%, jeweils bezogen auf den Katalysatorkreislauf und jeweils berechnet als metallisches Nickel(0), um die Aktivität des Hydrocyanierungskatalysators trotz Verlusten von Nickel(0)-Komplexen während der Reaktion in Schritt (a) oder während der Destillationsverfahren in Schritt (b) und (c), bevorzugt während der Reaktion in Schritt (a) sicherzustellen.

In dem erfindungsgemäßen Verfahren ist es besonders bevorzugt, wenn der Strom 5 an dem Seitenabzug in Verfahrensschritt (c) dampfförmig erhalten wird.

In einer weiteren bevorzugten Ausführungsform des erfindungsgemäßen Verfahrens ist es möglich, den Strom 1, der in Verfahrensschritt (a) erhalten wird, unter Ausschluss von Verfahrensschritt (b) direkt in Verfahrensschritt (c) zu überführen.

Der Strom 5 wird anschließend in ***Verfahrensschritt (d)*** in eine weitere Destillationsvorrichtung überführt. In dieser Destillationsvorrichtung erfolgt eine Destillation des Stromes 5 unter Erhalt eines Stromes 7, der 2-Methyl-3-butennitril enthält, und eines Stromes 8, der 3-Pentennitril enthält. Der Strom 7 wird am Kopf der Destillationsvorrichtung erhalten, während der Strom 8 im Sumpf der Destillationsvorrichtung erhalten wird.

Dabei wird in einer besonders bevorzugten Ausführungsform des erfindungsgemäßen Verfahrens der gegebenenfalls als gasförmiger Seitenabzug erhaltene Strom 5 gasförmig in die Destillationsvorrichtung von Verfahrensschritt (d) überführt, wobei der Druck an der Position der Zulaufstelle für Strom 5 in der Destillationsvorrichtung von Verfahrensschritt (d) kleiner oder gleich dem Druck an der Position des Seitenabzugs für Strom 5 in der Destillationsvorrichtung von Verfahrensschritt (c) ist.

Nicht ausgenommen vom Umfang dieser Beschreibung sind Verfahrensvarianten, bei denen der Druck der Stufe (d) frei gewählt wird und der Gasstrom 5 gegebenenfalls auf einen höheren Druck als an der Entnahmestelle in (c) verdichtet wird, um der Stufe (d) zugeführt werden zu können.

Der Verfahrensschritt (d) des erfindungsgemäßen Verfahrens kann in jeder geeigneten, dem Fachmann bekannten Apparatur durchgeführt werden. Für diese Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer einzigen Apparatur durchgeführt werden.

Die Kolonnen enthalten vorzugsweise strukturierte Packungen. Dabei erzeugen die strukturierten Packungen vorzugsweise 5 bis 100, besonders bevorzugt 10 bis 80, insbesondere 15 bis 50, theoretische Trennstufen.

Der Druck in Verfahrensschritt (d) beträgt vorzugsweise 0,001 bis 100 bar, besonders bevorzugt 0,01 bis 20 bar, insbesondere 0,05 bis 2 bar. Die Destillation wird so durchgeführt, dass die Temperatur im Sumpf der Destillationsvorrichtung vorzugsweise 30 bis 250 °C, besonders bevorzugt 50 bis 200 °C, insbesondere 60 bis 180 °C, beträgt. Die Destillation wird so durchgeführt, dass die Kondensationstemperatur am Kopf der Destillationsvorrichtung vorzugsweise -50 bis 250 °C, besonders bevorzugt 0 bis 180 °C, insbesondere 15 bis 160 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten

In einer Ausführungsform des erfindungsgemäßen Verfahrens kann Strom 7, der in dem Verfahrensschritt (d) erhalten wird, in den Verfahrensschritt (a) und/oder in Verfahrensschritt (b) zurückgeführt werden, wobei die Reaktionsbedingungen in Verfahrensschritt (a) oder die Verweilzeit der flüssigen Phase im Sumpf von Verfahrensschritt (b) so gewählt werden, dass 2-Methyl-3-butennitril zumindest teilweise zu trans-3-Pentennitril isomerisiert wird.

In einer weiteren Ausführungsform des erfindungsgemäßen Verfahrens wird der Strom 7 als Seitenabzugsstrom in der Destillationsvorrichtung des Verfahrensschritts (d) gewonnen, wobei als Kopfprodukt dieser Destillationskolonne ein Strom erhalten wird, der neben 2-Methyl-3-butennitril im Wesentlichem noch (Z)-2-Methyl-2-butennitril und gegebenenfalls 1,3-Butadien und Buten-Isomere sowie Vinylcyclohexen und Ethylidencyclohexen enthält.

Der Gehalt an trans-3-Pentennitril in dem Strom 7 beträgt vorzugsweise 0 bis 50 Gew.-%, besonders bevorzugt 100 Gew.-ppm bis 20 Gew.-%, insbesondere 1 bis 15 Gew.-%. Der Gehalt an 2-Methyl-3-butennitril in dem Strom 8 beträgt vorzugsweise 0 bis 10 Gew.-%, besonders bevorzugt 5 Gew.-ppm bis 5 Gew.%, insbesondere 50 Gew.-ppm bis 1 Gew.-%.

Das erfindungsgemäße Verfahren ermöglicht die Herstellung von 3-Pentennitril und 2-Methyl-3-butennitril in einem integrierten Verfahren, das aufgrund der nahezu vollständig möglichen Rückführung der 1,3-Butadienströme und des Katalysatorstroms eine hohe Verfahrensausbeute für die Einsatzstoffe aufweist. Dabei sind die zur destillativen Abtrennung von 1,3-Butadien und Pentennitril-Isomeren aus den katalysatorhaltigen Strömen nötigen Temperaturen und Druckverhältnisse so wählbar, dass einerseits die Sumpfverdampfertemperaturen bei Ausübung des Verfahrens im Produktionsmaßstab mit technisch erreichbaren Verweilzeiten so niedrig sind, dass sie vorzugsweise nicht zu einer Katalysatorschädigung führen und dass andererseits die Kondensation der Kopfprodukte der jeweiligen Destillationsschritte vorzugsweise bei Temperaturen stattfinden, bei denen die Wärmeabfuhr im Produktionsmaßstab mit wirtschaftlich vertretbarem Aufwand möglich ist.

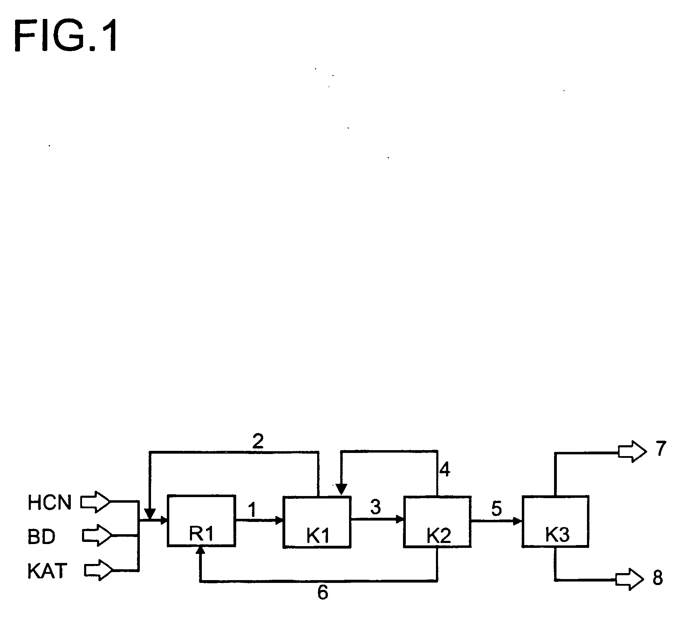

Eine Ausführungsform des erfindungsgemäßen Verfahrens wird anhand Figur 1 näher erläutert.

Figur 1 zeigt eine schematische Darstellung einer Ausführungsform des erfindungsgemäßen Verfahrens. In den Reaktor R1 wird 1,3-Butadien (BD), Cyanwasserstoff (HCN) und ein homogener Nickel(0)-Katalysator (KAT) eingeführt. In diesem Reaktor findet eine Hydrocyanierung von 1,3-Butadien statt. Hierbei bildet sich der Strom 1, der im Wesentlichen 3-Pentennitril, 2-Methyl-3-butennitril, den Nickel(0)-Katalysator, 1,3-Butadien und Cyanwasserstoff enthält. Dieser Strom wird anschließend in eine Destillationskolonne K1 überführt. Hier findet eine Auftrennung des Stromes 1 in einen Strom 2, der 1,3-Butadien enthält und in den Reaktor R1 zurückgeführt wird, und in einen Strom 3, der 3-Pentennitril, den Nickel(0)-Katalysator und 2-Methyl-3-butennitril enthält, statt.

Der Strom 3 wird anschließend in eine zweite Destillationskolonne K2 überführt. Hier erfolgt eine Abtrennung des restlichen 1,3-Butadiens (Strom 4) aus dem Strom 3 über Kopf der Kolonne, dessen Rückführung in Kolonne K1 sowie eine Abtrennung des Katalysators mit einem Strom 6 aus dem Sumpf der Kolonne, der an 3-Pentennitril und 2-Methyl-3-butennitril abgereichert ist. Strom 6 wird in Reaktor R1 zurückgeführt. An einem Seitenabzug der Kolonne K2 wird ein Strom 5 erhalten. Dieser Strom 5 enthält 3-Pentennitril und 2-Methyl-3-butennitril. Strom 4 wird in K1 zurückgeführt.

Der Strom 5 wird anschließend in eine dritte Destillationskolonne überführt K3. Hier erfolgt eine Auftrennung in den Strom 8, der 3-Pentennitril enthält und am Sumpf der Kolonne entnommen wird, und den Strom 7, der 2-Methyl-3-butennitril enthält und am Kopf der Destillationskolonne entnommen wird.

Der Strom 8, der 3-Pentennitril enthält, kann einer weiteren Hydrocyanierung zu Adipodinitril zugeführt werden.

Die vorliegende Erfindung wird anhand der nachfolgenden Ausführungsbeispiele näher erläutert.

In den Beispielen werden folgende Abkürzungen verwendet:

| | |
|---|---|
| HCN: | Cyanwasserstoff |
| KAT: | Katalysator |
| BD: | 1,3-Butadien |
| REG: | Regenerierungsstufe |

### Beispiel 1:

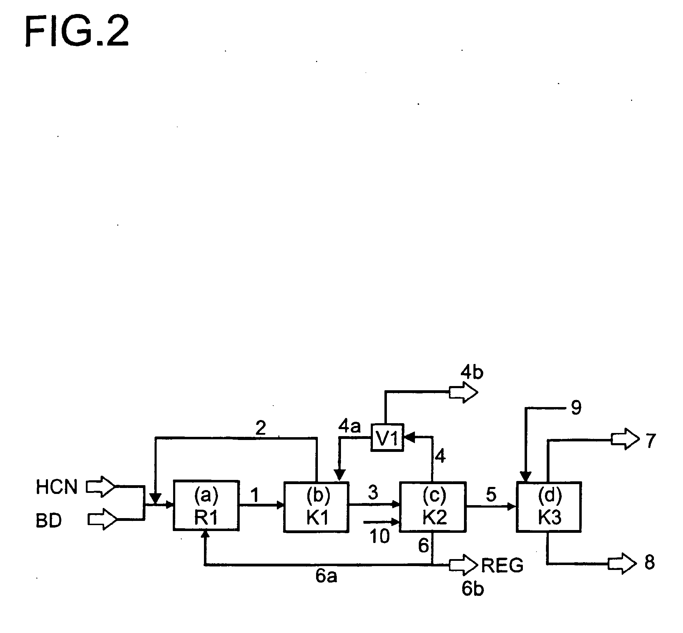

Beispiel 1 wird anhand Figur 2 verdeutlicht.

In Beispiel 1 wird für die Hydrocyanierung von 1,3-Butadien ein Katalysatorsystem auf Basis von Nickel(0)-Komplexen mit einem Gemisch von Liganden eingesetzt. Die Ligandmischung zur Hydrocyanierung enthält ca. 60 Mol% Tri(m/p-tolyl)phosphit und 40 Mol% des Chelatphosphonits 1:

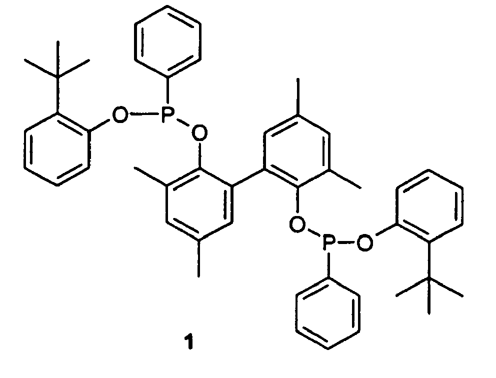

In einem Verfahrensschritt (a) werden folgende Ströme in einen Schlaufenreaktor R1 von 25 I Volumen gefahren, der mit einer Düse, Impulsaustauschrohr, externen Umpumpkreislauf und im einem im Umpumpkreislauf befindlichen Wärmetauscher zur Abfuhr der Reaktionsenergie ausgestattet und auf 357 K temperiert ist:
(1) 10 kg/h flüssiger, unstabilisierter und durch Destillation von Wasser befreiter Cyanwasserstoff;
(2) 22 kg/h handelsübliches mit tert.-Butylbrenzkatechin stabilisiertes 1,3-Butadien, enthaltend 0,25 Gew.-% cis-2-Buten, wobei das 1,3-Butadien durch Kontakt mit Aluminiumoxid behandelt wurde, um Wasser und den Stabilisator zu entfernen;
(3) 8 kg/h rückgeführtes 1,3-Butadien aus der Kolonne K1 des Verfahrensschritts (b) (Strom 2), so dass als gesamter 1,3-Butadien-Zulauf zum Reaktor R1 ein Strom von 30 kg/h, enthaltend 90 Gew.-% 1,3-Butadien, 5 Gew.-% cis-2-Buten sowie 5 Gew.-% 1-Buten erhalten wird;
(4) 21 kg/h Nickel(0)-Katalysatorlösung, erhalten wie in diesem Beispiel weiter unten beschrieben als Strom 6a aus der Kolonne (K2) des Verfahrensschritts (c).

Der aus dem Reaktor R1 abgezogene Strom 1 (63 kg/h) enthält in Summe 11 Gew.-% 1,3-Butadien und cis-2-Buten, entsprechend einem Umsatz von 79 % 1,3-Butadien, sowie in Summe 63 Gew.-% Pentennitrile, 31 Gew.-% trans-3-Pentennitril, 29 Gew.-% 2-Methyl-3-butennitril, untergeordnete Mengen cis-3-Pentennitril, trans-2-Pentennitril, cis-2-Pentennitril, 4-Pentennitril und geringe Mengen (Z)-2-Methyl-2-butennitril und (E)-2-Methyl-2-butennitril, sowie die Katalysatorbestandteile und Katalysatorabbauprodukte und Methylglutamitril.

Strom 1 wird in Verfahrensschritt (b) einer Destillationskolonne K1 zugeführt, die mit Verstärkungs- und Abtriebsteil betrieben wird und mit einem Fallfilmverdampfer und getrenntem Sumpf ausgestattet ist, sowie Kolonneneinbauten mit strukturierter Packung enthält, die 10 theoretische Trennstufen erzeugen. Kolonne K1 wird am Kopf mit einem Direktkondensator betriebenen, der aus einem mit strukturierter Packung bestückten Kolonnenschuss mit Totalfangtasse, Umpumpkreis und externen Wärmetauscher besteht. Die Kolonne K1 wird bei einem absoluten Druck von 2,0 bar Kopfdruck, 288 K Kopftemperatur und 363 K Sumpfabzugstemperatur betrieben.

Über Kopf der Kolonne K1 wird der Strom 2 erhalten, der wie eingangs beschrieben als Rückführstrom in den Reaktor R1 dosiert wird. Das Rücklaufverhältnis am Kopf der Kolonne K1 wird so eingestellt, dass der Strom 2 ca. 100 ppm 2-Methyl-3-butennitril enthält.

Über Sumpf der Kolonne K1 werden 59 kg/h eines Stromes 3 erhalten, der 2,9 Gew.-% 1,3-Butadien, 4,6 Gew.-% cis-2-Buten, 67 Gew.-% Pentennitrile sowie zusätzlich die Katalysatorbestandteile enthält. cis-2-Buten ist im Verhältnis zu 1,3-Butadien gegenüber dem Zulauf deutlich angereichert.

Strom 3 wird in einem Verfahrensschritt (c) in eine Destillationskolonne K2 gefahren, die in Abtriebsfahrweise betrieben wird und mit Fallfilmverdampfer, Kopfkondensator mit Nachkondensator sowie Kolonneneinbauten mit strukturierter Packung ausgestattet ist, die 10 theoretische Trennstufen erzeugen. Die Kolonne wird bei einem absoluten Druck von 150 mbar Kopfdruck, 329 K Kopftemperatur und 373 K Sumpfabzugstemperatur betrieben. Der Brüdenstrom der Kolonne wird bei 308 K teilkondensiert und mit einem Nachkondensator bei 263 K behandelt. Der so von 2-Methyl-3-butennitril und anderen Pentennitrilen abgereicherte Strom 4 wird in einem Verdichter V1 auf einen absoluten Druck von 1,2 bar verdichtet. Der verdichtete Gasstrom wird bei 279 K zum großen Teil unter Erhalt eines Stromes 4a (5 kg/h) kondensiert, wobei ein Teilstrom 4b (ca. 50 NI/h, enthaltend 44 Gew.-% cis-2-Buten) gasförmig entsorgt wird. Strom 4a wird flüssig in den Rücklaufteil des geteilten Sumpfes der Kolonne K1 zurückgefahren.

An der Kolonne K2 wird in einem gasförmigen Seitenabzug der Strom 5 gewonnen (40 kg/h), enthaltend ca. 50 ppm 1,3-Butadien, 46 Gew.-% 2-Methyl-3-butennitril und 48 Gew.-% trans-3-Pentennitril sowie in geringerem Umfang (E)-2-Methyl-2-butennitril und (Z)-2-Methyl-2-butennitril neben anderen Pentennitril-Isomeren. Die Position des Seitenabzugs ist so gewählt, dass unter dem Seitenabzug in einem Abtriebsteil die Komponente 2-Methyl-3-butennitril in dem über Sumpf gewonnen Strom 6 im Verhältnis zu trans-3-Pentennitril abgereichert wird.

In die Kolonne K2 werden 13 kg/h eines Katalysatorstromes (Strom 10) zusätzlich zu Strom 3 gefahren, enthaltend in Summe 73 Gew.-% Pentennitrile, 0,5 Gew.-% Ni(0), 18 Gew.-% Ligandmischung sowie ca. 5 Gew.-% Adipodinitril.

An der Kolonne K2 wird über Sumpf der Katalysator-Strom 6 erhalten, enthaltend 0,5 Gew.-% Ni(0), ca. 100 ppm 2-Methyl-3-butennitril und 35 Gew.-% restliche Pentennitrile. Der Strom 6 wird teilweise (Strom 6a) in den Reaktor R1 zurückgefahren (21 kg/h). Ein anderer Teil (Strom 6b: 5,4 kg/h) wird einer Regenerierung (REG) zugeführt, um nach der Regenerierung beispielsweise in Beispiel 1 der Hydrocyanierung von 3-Pentennitril gemäß der DE-A-102 004 004 683 eingesetzt zu werden.

Der Strom 5 wird in einem Verfahrensschritt (d) zu einer Destillationskolonne K3 gefahren, die mit Umlaufverdampfer und Kopfkondensator sowie mit einer strukturierten Packung ausgestattet ist, die 30 theoretische Trennstufen erzeugt. Die Kolonne K3 wird bei einem absoluten Druck von 180 mbar Kopfdruck, 345 K Kopftemperatur und 363 K Sumpfabzugstemperatur betrieben.

In die Kolonne K3 werden 39 kg/h eines Stromes 9 gefahren, enthaltend 54 Gew.-% trans-3-Pentennitril, 23 Gew.-% 2-Methyl-3-butennitril und 16 Gew.-% (Z)-2-Methyl-2-butennitril sowie in geringen Mengen weitere Pentennitril-Isomere. Strom 9 kann beispielsweise als rückgeführter Pentennitrilstrom aus einem Verfahren zur Isomerisierung von 2-Methyl-3-butennitril zu 3-Pentennitril erhalten werden, wie in Beispiel 1 der DE-A-102 004 004 671 beschrieben.

Über Kopf der Kolonne K3 werden 40 kg/h eines Stromes 7 erhalten, enthaltend 10 Gew.-% trans-3-Pentennitril, 68 Gew.-% 2-Methyl-3-butennitril, 16 Gew.-% (Z)-2-Methyl-2-butennitril sowie in Summe 0,1 Gew.-% 1,3-Butadien und cis-2-Buten. Dieser Strom kann einem Verfahren zur Isomerisierung von 2-Methyl-3-butennitril zu 3-Pentennitril zugeführt werden, wie in Beispiel 1 der DE-A-102 004 004 671 beschrieben.

Über Sumpf der Kolonne K3 werden 39 kg/h des Stromes 8 erhalten, enthaltend in Summe 97 Gew.-% trans-3-Pentennitril, cis-3-Pentennitril und 4-Pentennitril sowie ca. 100 ppm 2-Methyl-3-butennitril und ca. 1 Gew.-% (E)-2-Methyl-2-butennitril.

Beispiel 1 zeigt, wie eine nahezu vollständige Rückgewinnung von Butadien und Katalysator in einem Hydrocyanierungsverfahren gelingt. In Beispiel 1 wird der Katalysator unter schonenden Bedingungen in Kolonne K1 und Kolonne K2 zweistufig abgetrennt, wobei in der Kolonne K2 der Nitrilstrom im Wesentlichen frei von Butadien gewonnen werden kann.

Die in Beispiel 1 vorgefundene Zusammensetzung des zurückgewonnen Katalysatorstroms ist für den Einsatz in einem Verfahren zur Hydrocyanierung von Pentennitril zu Adipodinitril besonders geeignet, da Strom 6 und somit auch Strom 6b im Wesentlichen frei von 2-Methyl-3-butennitril und Butadien erhalten wird.

### Beispiel 2:

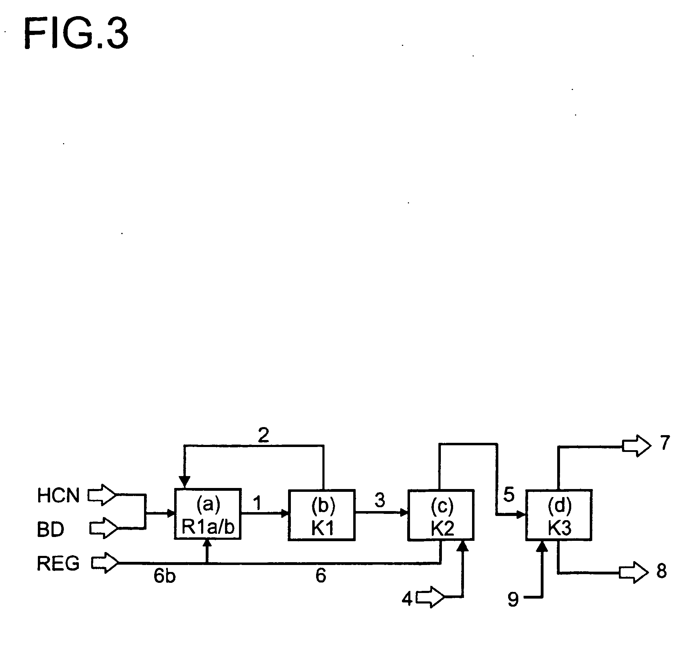

Beispiel 2 wird anhand Figur 3 verdeutlicht.

In Beispiel 2 wird für die Hydrocyanierung von 1,3-Butadien ein Katalysatorsystem auf

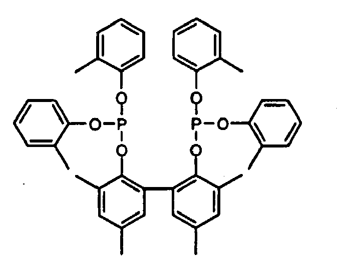

Basis von Nickel(0)-Komplexen mit Chelatphosphit 2 als Ligand verwendet:
In einem Verfahrensschritt (a) werden folgende Ströme in ein System aus zwei Reaktoren R1a und R1b von je 12 I Volumen gefahren, die jeweils mit einer Düse, Impulsaustauschrohr, externen Umpumpkreislauf und in einem im Umpumpkreislauf befindlichen Wärmetauscher zur Abfuhr der Reaktionsenergie ausgestattet sind und auf 363 K temperiert sind: **2**
   (1) 6 kg/h flüssiger, unstabilisierter, durch Destillation von Wasser befreiter Cyanwasserstoff zu R1a;
   (2) 6 kg/h flüssiger, unstabilisierter, durch Destillation von Wasser befreiter Cyanwasserstoff zu R1b;
   (3) 25 kg/h handelsübliches 1,3-Butadien zu R1a, enthaltend 0,25 Gew.-% cis-2-Buten, wobei das 1,3-Butadien durch Kontakt mit Aluminiumoxid behandelt wurde, um Wasser und den Stabilisator zu entfernen;
   (4) 2 kg/h rückgeführtes 1,3-Butadien aus Kolonne K1 in Verfahrensschritt (b) zu R1a (Strom 2), so dass als gesamter 1,3-Butadien-Zulauf zum Reaktor R1 ein Strom von 27 kg/h, enthaltend 98 Gew.-% 1,3-Butadien und in Summe 2 Gew.-% cis-2-Buten und 1-Buten erhalten wird;
   (5) 14 kg/h Nickel(0)-Katalysatorlösung zu R1a, erhalten wie in diesem Beispiel weiter unten beschrieben als Strom 6a aus der Kolonne (K2) des Verfahrensschritts (c).

Der aus dem Reaktor R1b abgezogene Strom 1 (54 kg/h) enthält in Summe 4 Gew.-% 1,3-Butadien und cis-2-Buten, entsprechend einem Umsatz von 94 % 1,3-Butadien, sowie in Summe 74 Gew.-% Pentennitrile, davon 33 Gew.-% trans-3-Pentennitril, 37 Gew.-% 2-Methyl-3-butennitril, untergeordnete Mengen cis-3-Pentennitril, trans-2-Pentennitril, cis-2-Pentennitril, 4-Pentennitril und geringe Mengen (Z)-2-Methyt-2-butennitril und (E)-2-Methyl-2-butennitril, sowie die Katalysatorbestandteile und Katalysatorabbauprodukte und Methylglutamitril.

Strom 1 wird in einem Verfahrensschritt (b) in einer Destillationskolonne K1 zugeführt, die als Verstärkungskolonne betrieben wird und mit einem Fallfilmverdampfer ausgestattet ist sowie Kolonneneinbauten mit strukturierter Packung enthält, die 4 theoretische Trennstufen erzeugen. Kolonne K1 wird am Kopf mit einem Direktkondensator betriebenen, der aus einem mit Füllkörperschüttung bestückten Kolonnenschuss mit Totalfangtasse, Umpumpkreis und externen Wärmetauscher besteht. Die Kolonne K1 wird bei einem absoluten Druck von 0,8 bar Kopfdruck, 263 K Kopftemperatur und 393 K Sumpfabzugstemperatur betrieben.

Über Kopf der Kolonne K1 wird der Strom 2 erhalten, der wie eingangs beschrieben als Rückführstrom in den Reaktor R1a dosiert wird. Das Rücklaufverhältnis am Kopf der Kolonne K1 wird so eingestellt, dass der Strom 2 0,1 Gew.-% 2-Methyl-3-butennitril enthält.

Über Sumpf der Kolonne K1 werden 52 kg/h eines Stromes 3 erhalten, der 0,3 Gew.-% 1,3-Butadien, 0,1 Gew.-% cis-2-Buten, 76 Gew.-% Pentennitrile sowie zusätzlich die Katalysatorbestandteile enthält.

Strom 3 wird in Verfahrensschritt (c) in eine Destillationskolonne K2 gefahren, die in Abtriebsfahrweise betrieben wird und mit Fallfilmverdampfer, Kopfkondensator mit Nachkondensator sowie mit Kolonneneinbauten mit strukturierter Packung ausgestattet ist, die 4 theoretische Trennstufen erzeugen. Die Kolonne wird bei einem absoluten Druck von 70 mbar Kopfdruck, 333 K Kopftemperatur und 373 K Sumpfabzugstemperatur betrieben.

An der Kolonne K2 wird der gasförmige Kopfabzug Strom 5 gewonnen (40 kg/h), enthaltend 0,4 Gew.-% 1,3-Butadien, 54 Gew.-% 2-Methyl-3-butennitril und 42 Gew.-% trans-3-Pentennitril sowie in geringerem Umfang (E)-2-Methyl-2-butennitril und (Z)-2-Methyl-2-butennitril neben anderen Pentennitril-Isomeren.

In die Kolonne K2 werden 3 kg/h eines Katalysatorstromes (Strom 4) gefahren, enthaltend in Summe 45 Gew.-% Pentennitrile, 1,5 Gew.-% Ni(0) und den Chelatligand, erhalten beispielsweise durch Umsetzung von Nickel(0)(Cyclooctadienyl)₂-Komplex mit dem Chelatphosphit 2.

An der Kolonne K2 wird über Sumpf der Katalysator-Strom 6 erhalten, enthaltend 1,2 Gew.-% Ni(0), 0,3 Gew.-% 2-Methyl-3-butennitril und 17 Gew.-% restliche Pentennitrile. Der Strom 6 wird teilweise (Strom 6a) in den Reaktor R1 zurückgefahren (14 kg/h). Ein anderer Teil (Strom 6b: 3,8 kg/h) wird einer Regenerierung (REG) zugeführt und kann nach der Regenerierung (REG) beispielsweise in der Hydrocyanierung von 3-Pentennitril eingesetzt oder gegebenenfalls in die Hydrocyanierung von 1,3-Butadien gemäß dem erfindungsgemäßem Verfahren zurückgeführt werden.

Der Strom 5 wird in einem Verfahrensschritt (d) zu einer Destillationskolonne K3 gefahren, die mit Umlaufverdampfer und Kopfkondensator sowie mit einer strukturierten Packung ausgestattet ist, die 45 theoretische Trennstufen erzeugt. Die Kolonne K3 wird bei einem absoluten Druck von 1,0 bar Kopfdruck, 395 K Kopftemperatur und 416 K Sumpfabzugstemperatur betrieben.

In die Kolonne K3 werden 24 kg/h Rückführstrom 9 zugefahren, enthaltend 70 Gew.-% trans-3-Pentennitril, 14 Gew.-% 2-Methyl-3-butennitril und 7 Gew.-% (Z)-2-Methyl-2-butennitril sowie in geringen Mengen weitere Pentennitril-Isomere. Strom 9 kann beispielsweise als rückgeführter Pentennitrilstrom aus einem Verfahren zur Isomerisierung von 2-Methyl-3-butennitril zu 3-Pentennitril erhalten werden, wie in Beispiel 2 der DE-A-102 004 004 671 beschrieben.

Über Kopf der Kolonne K3 werden 30 kg/h eines Stromes 7 erhalten, enthaltend 1 Gew.-% trans-3-Pentennitril, 85 Gew.-% 2-Methyl-3-butennitril, 8 Gew.-% (Z)-2-Methyl-2-butennitril sowie in Summe 3 Gew.-% 1,3-Butadien und cis-2-Buten. Das Rücklaufverhältnis der Kolonne K3 wird so eingestellt, dass über Kopf 1 Gew.-% 3-Pentennitril erhalten wird. Dieser Strom kann beispielsweise einem Verfahren zur Isomerisierung von 2-Methyl-3-butennitril zu 3-Pentennitril zugeführt werden, wie in Beispiel 2 der DE-A-102 004 004 671 beschrieben.

Über Sumpf der Kolonne K3 werden 38 kg/h des Stromes 8 erhalten, enthaltend in Summe 97 Gew.-% trans-3-Pentennitril, cis-3-Pentennitril trans-2-Pentennitril, cis-2-Pentennitril und 4-Pentennitril sowie ca. 10 ppm 2-Methyl-3-butennitril und ca. 2 Gew.-% (E)-2-Methyl-2-butennitril und in geringen Mengen Methylglutamitril. Strom 8 kann einem Verfahren zur Hydrocyanierung von 3-Pentennitril zu Adipodinitril zugeführt werden, wie in Beispiel 2 der DE-A-102 004 004 683 beschrieben.

In Beispiel 2 wird der Strom 6 in der Kolonne K2 und somit auch Strom 6b ohne die entsprechenden Trennstufen mit einem merklichen Anteil an 2-Methyl-3-butennitril erhalten (ca. 1.5 Gew.-% bezogen auf den Nitrilgehalt des Katalysatorstromes in Beispiel 2 anstelle ca. 0,1 Gew.-% in Beispiel 1), der zu einem merklichen Wertproduktverlust durch Bildung von Methylglutamitril führt, wenn dieser Katalysator nach der Regenerierung zur Hydrocyanierung von 3-Pentennitril zu Adipodinitril eingesetzt wird.

### Beispiel 3:

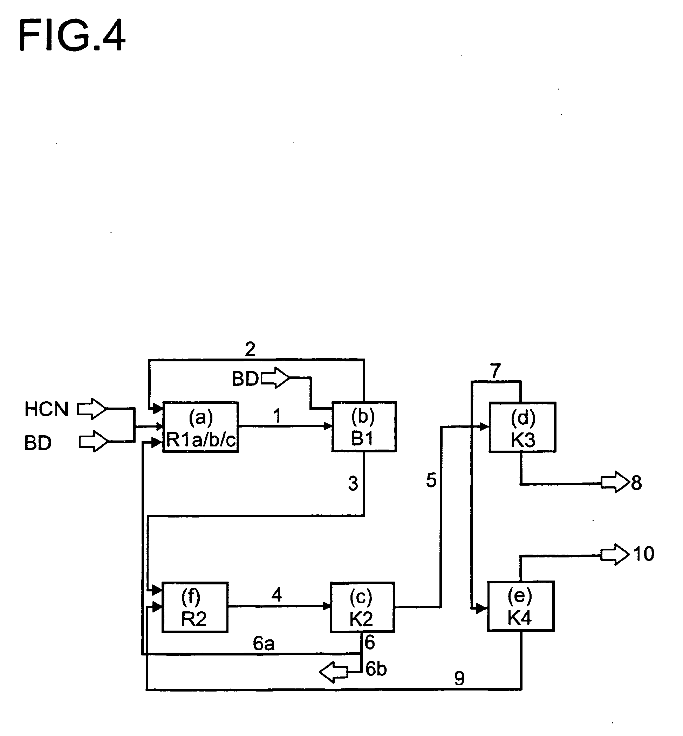

Beispiel 3 wird anhand Figur 4 verdeutlicht.

In Beispiel 3 wird für die Hydrocyanierung von 1,3-Butadien ein Katalysatorsystem auf Basis von Nickel(0)-Komplexen mit einem Gemisch von Liganden eingesetzt. Die Ligandmischung zur Hydrocyanierung enthält ca. 80 Mol% Tri(m/p-tolyl)phosphit und 20 Mol% des Chelatphosphits 2.

In einem Verfahrensschritt (a) werden folgende Ströme in ein System aus drei hintereinandergeschalteten kontinuierlich betriebenen Rührkesseln R1a, R1b und R1c von je 10 I Volumen gefahren, die auf 373 K temperiert sind:
(1) 5,2 kg/h flüssiger, unstabilisierter, durch Destillation von Wasser befreiter Cyanwasserstoff zu R1a;
(2) 4,0 kg/h flüssiger, unstabilisierter, durch Destillation von Wasser befreiter Cyanwasserstoff zu R1b;
(3) 23 kg/h 1,3-Butadien als Strom 2 vom Kondensator des Verdampfers B1 in Verfahrensschritt (b), enthaltend 92 Gew.-% 1,3-Butadien, 2 Gew.-% trans-3-Pentennitril, 4 Gew.-% 2-Methyl-3-butennitril und ca. 2 Gew.-% cis-2-Buten zu R1a;
(4) 4,1 kg/h Nickel(0)-Katalysatorlösung zu R1a, erhalten wie in diesem Beispiel weiter unten beschrieben als Strom 6a aus der Rektifikationskolonne K2 in Verfahrensschritt (c);
(5) 3,7 kg/h Nickel(0)-Katalysatorlösung zu R1a (KAT), enthaltend in Summe 45 Gew.-% Pentennitrile, 1,1 Gew.-% Ni(0), 38 Gew.-% Ligandmischung sowie ca. 12 Gew.-% Adipodinitril.

Der Reaktor R1c wird als Nachreaktor mit dem Ablauf aus Reaktor R1b bei 353 K betrieben.

Der aus dem Reaktor R1c abgezogene Strom 1 (37 kg/h) enthält 7 Gew.-% 1,3-Butadien, entsprechend einem Umsatz von 86 % 1,3-Butadien, sowie in Summe 77 Gew.-% Pentennitrile, davon 33 Gew.-% trans-3-Pentennitril, 41 Gew.-% 2-Methyl-3-butennitril, untergeordnete Mengen cis-3-Pentennitril, trans-2-Pentennitril, cis-2-Pentennitril, 4-Pentennitril und geringe Mengen (Z)-2-Methyl-2-butennitril und (E)-2-Methyl-2-butennitril, sowie die Katalysatorbestandteile, Katalysatorabbauprodukte und Methylglutarnitril.

Strom 1 wird in einem Verfahrensschritt (b) in einer Verdampferstufe B1 zugeführt, die mit einem Umlaufverdampfer ausgestattet ist. Die Verdampferstufe B1 wird am Kopf mit einem Kondensator betrieben, der mit kondensiertem Material aus dem Rücklaufbehälter gespült wird. Die Verdampferstufe B1 wird bei einem absoluten Druck von 0,5 bar Kopfdruck, 253 K Kondensationstemperatur und 363 K Sumpfabzugstemperatur betrieben.

In den Kondensatsammelbehälter der Verdampferstufe B1 werden 18,5 kg/h handelsübliches 1,3-Butadien dosiert, enthaltend 0,25 Gew.-% cis-2-Buten, das durch Kontakt mit Molsieb behandelt wurde, wobei der Wassergehalt des eingesetzten 1,3-Butadiens auf kleiner 10 Gew.-ppm H₂O verringert wurde.

Aus dem Kondensatsammelbehälter der Verdampferstufe B1 wird Strom 2 als Summe von rückgeführtem und frisch zudosiertem 1,3-Butadien abgezogen und zum Reaktor R1a, wie vorher beschrieben, zurückgeführt.

Über den Sumpf der Verdampferstufe B1 werden 37 kg/h eines Stromes 3 erhalten, der in Summe 1 Gew.-% 1,3-Butadien und cis-2-Buten, 82 Gew.-% Pentennitrile sowie zusätzlich die Katalysatorbestandteile enthält.

Strom 3 wird in einem Verfahrensschritt (f) in einen auf 383 K temperierten Reaktor R2, ausgeführt als Rührkessel mit nachgeschalteter Verweilzeitstrecke gefahren, wobei 2-Methyl-3-butennitril in Gegenwart des Nickelkatalysators und einer Lewis-Säure zu trans-3-Pentennitril isomerisiert wird.

In den Reaktor R2 wird ein Pentennitril-Rückführstrom 9 gefahren (10 kg/h), der in Verfahrensschritt (e) in Kolonne 4 als Sumpfprodukt erhalten wird, enthaltend 60 Gew.-% 2-Methyl-3-butennitril, in Summe 10 Gew.-% trans-3-Pentennitril mit weiteren Pentennitrilisomeren sowie Vinylcyclohexen und in geringen Mengen 1,3-Butadien.

Aus Reaktor R2 wird ein Strom 4 erhalten (45 kg/h), enthaltend 62 Gew.-% trans-3-Pentennitril und 14 Gew.-% 2-Methyl-3-butennitril, entsprechend einem Umsatz von 70 Gew.-% 2-Methyl-3-butennitril zu trans-3-Pentennitril, sowie die Katalysatorkomponenten.

Strom 4 wird in einem Verfahrensschritt (c) in eine Rektifikationskolonne K2 gefahren, die mit Fallfilmverdampfer und Kondensator ausgestattet ist und bei einem absoluten Druck von 50 mbar und 393 K Sumpfabzugstemperatur als Abtriebskolonne mit Kolonneneinbauten betrieben wird, die 10 destillative Trennstufen zur Verfügung stellen.

Aus dem Kondensator der Rektifikationskolonne K2 wird ein Strom 5 gewonnen (38 kg/h), enthaltend 91 Gew.-% Pentennitrilisomere sowie ca. 1 Gew.-% 1,3-Butadien und in geringerem Umfang (E)-2-Methyl-2-butennitril, (Z)-2-Methyl-2-butennitril und Vinylcyclohexen.

An der Rektifikationskolonne K2 wird über Sumpf der Katalysator-Strom 6 erhalten (7 kg/h), enthaltend 1,3 Gew.-% Ni(0), ca. 20 ppm 2-Methyl-3-butennitril, 17 Gew.-% restliche Pentennitrile, die restlichen Katalysatorbestandteile, Adipodinitril und Methylglutarnitril. Der Strom 6 wird teilweise (Strom 6a) in den Reaktor R1 zurückgefahren (4,4 kg/h). Der Rest (Strom 6b) kann einer Regenerierung (REG) zugeführt, und anschließend beispielsweise in einer Hydrocyanierung von 3-Pentennitril eingesetzt werden (entsprechend US 2003/0100442 oder entsprechend DE-A-103 51 002. Darüber hinaus kann der Katalysator im erfindungsgemäßen Verfahren zur Hydrocyanierung von 1,3-Butadien, gegebenenfalls nach Abtrennung von Zinkchlorid, wieder eingesetzt werden.

Der Strom 5 wird in einem Verfahrensschritt (d) zu einer Destillationskolonne K3 gefahren, die mit Zwangsumlaufverdampfer und Kopfkondensator sowie mit Kolonneneinbauten ausgestattet ist, die 30 theoretische Trennstufen erzeugen. Die Kolonne K3 wird bei einem absoluten Druck von 0,12 bar Kopfdruck, 334 K Kopftemperatur und 352 K Sumpfabzugstemperatur betrieben.

Über Kopf der Kolonne K3 werden 10 kg/h eines Stromes 7 erhalten, enthaltend 5 Gew.-% trans-3-Pentennitril, 60 Gew.-% 2-Methyl-3-butennitril, 4 Gew.-% (Z)-2-Methyl-2-butennitril sowie in Summe 4 Gew.-% 1,3-Butadien und cis-2-Buten. Das Rücklaufverhältnis der Kolonne K3 wird so eingestellt, dass über Kopf 5 Gew.-% 3-Pentennitril erhalten werden.

Über Sumpf der Kolonne K3 werden 27 kg/h des Stromes 8 erhalten, enthaltend in Summe 98 Gew.-% trans-3-Pentennifil, cis-3-Pentennitril und 4-Pentennitril sowie ca. 1000 ppm 2-Methyl-3-butennitril und ca. 2 Gew.-% (E)-2-Methyl-2-butennitril.

Strom 7 wird in einem Verfahrensschritt (e) in eine Destillationskolonne K4 gefahren, die als Verstärkungskolonne betrieben wird und mit Zwangsumlaufverdampfer, Kopfkondensator, Rücklaufteiler, sowie Kolonneneinbauten mit strukturierter Packung ausgestattet ist, die 15 theoretische Trennstufen erzeugen. Die Kolonne K4 wird bei einem absluten Druck von 380 mbar Kopfdruck, 361 K Kopftemperatur und 365 K Sumpfabzugstemperatur betrieben.

In Kolonne K4 wird über Kopf ein flüssiger Strom 10 erhalten (0,6 kg/h), enthaltend in Summe 4 Gew.-% 1,3-Butadien und cis-2-Buten, 54 Gew.-% 2-Methyl-3-butennitril, 38 Gew.-% (Z)-2-Methyl-2-butennitril sowie 2,5 Gew.-% Vinylcyclohexen. Die Abzugsmenge von Strom 10 vom Kopf der Kolonne K4 wird so eingestellt, dass am Kopfabzugsstrom 7 der Kolonne K3 in Summe 30 Gew.-% (Z)-2-Methyl-2-butennitril und Vinylcyclohexen enthalten sind. In Kolonne K4 wird am als Teilkondensator betriebenen Kopfkondensator ein gasförmiger Strom erhalten (195 Norm-I/h), der im Wesentlichen 1,3-Butadien enthält.

In Kolonne K4 wird über Sumpf der Strom 9 erhalten (9,4 kg/h), der neben 3-Pentennitrilen im Wesentlichen das in der Isomerisierung nicht umgesetzte 2-Methyl-3-butennitril enthält und in den Isomerisierungsreaktor R2 in Schritt (f) zurückgefahren wird.

In Beispiel 3 wird die Destillationsvorrichtung K1 aus Beispiel 1 als einstufige Verdampfung B1 ausgeführt, was im Vergleich zu Beispiel 1 zu einer deutlich höheren Konzentration von Nitrilen, insbesondere 2-Methyl-3-butennitril, im rückgeführten Butadien und zu höheren Butadienverlusten führt.

### Beispiel 4:

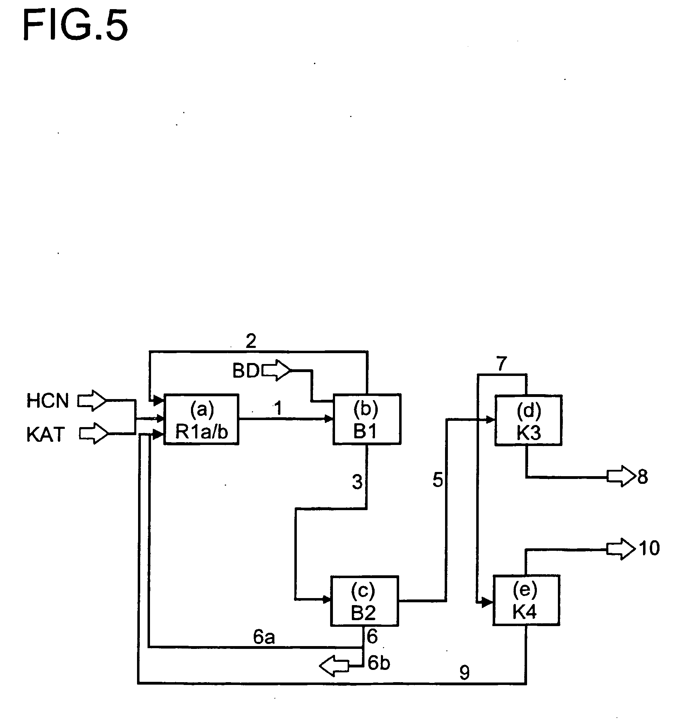

Beispiel 4 wird anhand Figur 5 verdeutlicht.

In .Beispiel 4 wird für die Hydrocyanierung von 1,3-Butadien ein Katalysatorsystem auf Basis von Nickel(0)-Komplexen mit einem Gemisch von Liganden eingesetzt. Die Ligandmischung zur Hydrocyanierung enthält ca. 80 Mol% Tri(m/p-tolyl)phosphit und 20 Mol% des Chelatphosphonits 1.

In einem Verfahrensschritt (a) werden folgende Ströme in ein System aus zwei hintereinandergeschalteten kontinuierlich betriebenen Rührkesseln R1a und R1b von jeweils 50 I Volumen gefahren, die auf 363 K temperiert sind:
(1) 18 kg/h flüssiger, unstabilisierter, durch Destillation von Wasser befreite Cyanwassserstoff zugleichen Teilen auf die Reaktoren R1a und R1b,
(2) 62 kg/h 1,3-Butadien als Strom 2 vom Kopf des Verdampfers B1 in Verfahrensschritt (b), enthaltend 87 Gew.-% 1,3-Butadien, 3 Gew.-% trans-3-Pentennitril, 6 Gew.-% 2-Methyl-3-butennitril und ca. 2 Gew.-% cis-2-Buten zu Reaktor R1a.
(3) 61 kg/h Nickel(0)-Katalysatorlösung, erhalten wie in diesem Beispiel weiter unten beschrieben als Strom 6a aus der Verdampferstufe B2 in Verfahrensschritt (c) zu Reaktor R1a,
(4) 6,7 kg/h Nickel(0)-Katalysatorlösung zu R1a (KAT), erhalten wie in Beispiel 1 der DE-A-102 004 004 683 beschrieben als Sumpfabzug der Kolonne K4 aus Verfahrensschritt (4) von Beispiel 2 aus dieser Patentanmeldung erhalten wird, enthaltend in Summe 45 Gew.-% Pentennitrile, 1,1 Gew.-% Ni(0), 38 Gew.-% Ligandmischung sowie ca. 12 Gew.-% Adipodinitril zu Reaktor R1a, wobei der 1,3-Butadien-Strom und der Katalysatorstrom vor dem Kontaktieren mit Cyanwasserstoff vorgemischt werden,
(5) 29 kg/h eines Nitril-Rückführstromes 9 erhalten als Sumpfabzug der Kolonne K4 wie weiter unten im Beispiel beschrieben, enthaltend 19 Gew.-% trans-3-Pentennitril, 62 Gew.-% 2-Methyl-3-butennnitril, weitere Nitrile und Vinylcyclohexen.

Der aus dem Reaktor R1b abgezogene Strom 1 (177 kg/h) enthält 11 Gew.-% 1,3-Butadien, entsprechend einem Umsatz von 66 % 1,3-Butadien, sowie in Summe 64 Gew.-% Pentennitrile, davon 32 Gew.-% trans-3-Pentennitril, 30 Gew.-% 2-Methyl-3-butennitril, untergeordnete Mengen cis-3-Pentennitril, trans-2-Pentennitril, cis-2-Pentennitril, 4-Pentennitril und geringe Mengen (Z)-2-Methyl-2-butennitril und (E)-2-Methyl-2-butennitril, sowie die Katalysatorbestandteile und Katalysatorabbauprodukte.

Strom 1 wird in einem Verfahrensschritt (b) in einer Verdampferstufe B1 zugeführt, die mit einem Fallfilmverdampfer ausgestattet ist. Die Verdampferstufe B1 wird am Kopf mit einem Kondensator betriebenen, der mit kondensierten Material aus dem Rücklaufbehälter gespült wird. Die Verdampferstufe B1 wird bei einem absoluten Druck von 1,3 bar Kopfdruck, 278 K Kondensationstemperatur und 403 K Sumpfabzugstemperatur betrieben.

In den Kondensatsammelbehälter der Verdampferstufe B1 werden 37 kg/h handelsübliches 1,3-Butadien dosiert, enthaltend 0,25 Gew.% cis-2-Buten, das durch Kontakt mit Molsieb behandelt wurde, wobei der Wassergehalt vom eingesetzten 1,3-Butadien auf weniger als 5 Gew.-ppm reduziert wurde und wobei der im eingesetzten 1,3-Butadien enthaltene Stabilisator tert.-Butylbrenzkatechin in Konzentrationen im ppm-Maßstab in den Kondensatsammelbehälter und den Kondensator-Spülkreislauf gelangt.

Aus dem Kondensatsammelbehälter der Verdampferstufe B1 wird Strom 2 als Summe von rückgeführtem und frisch zudosiertem 1,3-Butadien abgezogen und zum Reaktor R1a, wie vorher beschrieben, zurückgeführt.

Über Sumpf der Verdampferstufe B1 werden 152 kg/h eines Stromes 3 erhalten, der 0,9 Gew.% 1,3-Butadien, 16 Gew.% 2-Methyl-3-butennitril, 51 Gew.-% trans-3-Pentennitril und weitere Pentennitrilisomere sowie zusätzlich die Katalysatorbestandteile enthält. Die Zusammensetzung des Sumpfaustrags der Verdampferstufe lässt auf einen Umsatzgrad von 50 Gew.-% 2-Methyl-3-butennitril zu trans-3-Pentennitril im Sumpf des Verdampfers schliessen.

Strom 3 wird in einem Verfahrensschritt (c) in eine Verdampferstufe B2 gefahren, die mit Fallfilmverdampfer und Kondensator ausgestattet ist und bei einem absoluten Druck von 260 mbar und 383 K Sumpfabzugstemperatur betrieben wird.

Aus der Verdampferstufe B2 wird ein Strom 5 gasförmig gewonnen (83 kg/h), enthaltend 93 Gew.% Pentennitrilisomere, ca. 1 Gew.% 1,3-Butadien und in geringerem Umfang (E)-2-Methyl-2-butennitril, (Z)-2-Methyl-2-butennitril und Vinylcyclohexen. Strom 5 wird in die Destillationskolonne K3 in Verfahrensschritt (d) gefahren.

An der Verdampferstufe B2 wird über Sumpf der Katalysator-Strom 6 erhalten (69 kg/h), enthaltend 0,6 Gew.-% Ni(0), 2 Gew.-% 2-Methyl-3-butennitril und 42 Gew.-% restliche Pentennitrile. Der Strom 6 wird größtenteils (Strom 6a) in den Reaktor R1 zurückgefahren (61,4 kg/h). Der Rest (Strom 6b) wird einer Regenerierung (REG), beispielsweise gemäß der DE-A-103 51 002, zugeführt und kann in der Hydrocyanierung von 3-Pentennitril, beispielsweise gemäß der DE-A-102 004 004 eingesetzt werden.

Der Strom 5 wird in einem Verfahrensschritt (d) gasförmig zu einer Destillationskolonne K3 gefahren, die mit Zwangsumlaufentspannungsverdampfer und Kopfkondensator sowie mit einer strukturierten Packung ausgestattet ist, die 30 theoretische Trennstufen erzeugt. Die Kolonne K3 wird bei einem absoluten Druck von 80 mbar Kopfdruck, 375 K Kopftemperatur und 343 K Sumpfabzugstemperatur betrieben.

Über Kopf der Kolonne K3 werden 36 kg/h eines Strom 7 erhalten, enthaltend 15 Gew.% trans-3-Pentennitril, 64 Gew.-% 2-Methyl-3-butennitril, 3 Gew.-% (Z)-2-Methyl-2-butennitril sowie in Summe 4 Gew.% 1,3-Butadien und cis-2-Buten. Das Rücklaufverhältnis der Kolonne K3 wird so eingestellt, dass über Kopf 15 Gew.% trans-3-Pentennitril erhalten werden.

Über Sumpf der Kolonne K3 werden 47 kg/h des Stromes 8 erhalten, enthaltend in Summe 98 Gew.% trans-3-Pentennitril, cis-3-Pentennitril, trans-2-Pentennitril, cis-2-Pentennitril und 4-Pentennitril sowie 100 ppm 2-Methyl-3-butennitril und ca. 1 Gew.-% (E)-2-Methyl-2-butennitril.

Strom 7 wird in einem Verfahrensschritt (e) in eine Destillationskolonne K4 gefahren, die als Verstärkungskolonne betrieben wird und mit Zwangsumlaufverdampfer, Kopfkondensator, Rücklaufteiler, sowie Kolonneneinbauten mit strukturierter Packung ausgestattet ist, die 45 theoretische Trennstufen erzeugen. Die Kolonne wird bei einem absoluten Druck von 320 mbar Kopfdruck, 288 K Kondensationstemperatur und 363 K Sumpfabzugstemperatur betrieben.

In dieser Kolonne K4 wird über Kopf ein flüssiger Strom 10 erhalten (6,8 kg/h), enthaltend in Summe 10 Gew.-% 1,3-Butadien und cis-2-Buten, 80 Gew.% 2-Methyl-3-butennitril, 8 Gew.% (Z)-2-Methyl-2-butennitril, sowie 0,5 Gew.-% Vinylcyclohexen. In Kolonne K4 wird am als Teilkondensator betriebenen Kopfkondensator ein gasförmiger Strom erhalten (ca. 250 Norm-l/h), der im wesentlichen 1,3-Butadien enthält.

In Kolonne K4 wird über Sumpf der Strom 9 erhalten (28,7 kg/h), der neben 3-Pentennitrilen im Wesentlichen das in der Isomerisierung nicht umgesetzte 2-Methyl-3-butennitril enthält und in den Hydrocyanierungsreaktor R1 zurückgefahren wird.

In Beispiel 4 wird sowohl die Destillationsvorrichtung K1 als auch die Destillationsvorrichtung K2 aus dem Beispiel 1 jeweils als einstufige Verdampferstufen B1 und B2 ausgeführt, was im Vergleich zu Beispiel 1 auch bei Anpassung der Verhältnisse in der Stufe B1 zu merklichen Butadien-Verlusten führt und den Katalysatorstrom thermisch stärker als in Beispiel 1 belastet.

### Beispiel 5:

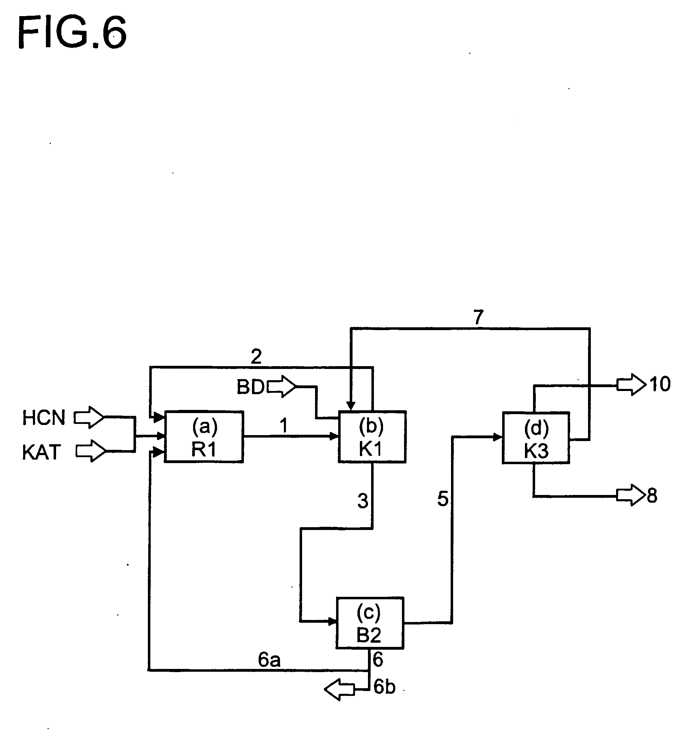

Beispiel 5 wird anhand Figur 6 verdeutlicht.

In Beispiel 5 wird für die Hydrocyanierung von 1,3-Butadien ein Katalysatorsystem auf Basis von Nickel(0)-Komplexen mit Chelatphosphonit 1 als Ligand eingesetzt.

In einem Verfahrensschritt (a) werden folgende Ströme in ein einen kontinuierlich betriebenen Rührkessel R1 von 30 I Volumen gefahren, der auf 363 K temperiert ist:
(1) 16 kg/h flüssiger, unstabilisierter, durch Destillation von Wasser befreite Cyanwasserstoff,
(2) 50 kg/h 1,3-Butadien als Strom 2 vom Kopf des Verdampfers B1 in Verfahrensschritt (b), enthaltend 94 Gew.% 1,3-Butadien, 2 Gew.-% trans-3-Pentennitril, 4 Gew.-% 2-Methyl-3-butennitril und ca. 1 Gew.-% cis-2-Buten,
(3) 10 kg/h Nickel(0)-Katalysatorlösung, erhalten wie in diesem Beispiel weiter unten beschrieben als Strom 6a aus der Verdampferstufe B2 in Verfahrensschritt (c), enthaltend in Summe 42 Gew.-% Pentennitrile, 23 Gew.% Ligand, 0,9 Gew.% Nickel(0), sowie jeweils ca. 10 Gew.-% Adipodinitril und Methylglutarnitril,
(4) 4 kg/h Nickel(0)-Katalysatorlösung zu R1 (KAT), enthaltend in Summe 45 Gew.-% Pentennitrile, 1,5 Gew.-% Ni(0) und 48 Gew.% Ligand.

Der aus dem Reaktor R1 abgezogene Strom 1 (89 kg/h) enthält 17 Gew.-% 1,3-Butadien, entsprechend einem Umsatz von 71 % 1,3-Butadien, sowiein Summe 73 Gew.-% Pentennitrile, davon 32 Gew.-% trans-3-Pentennitril, 36 Gew.-% 2-Methyl-3-butennitril, untergeordnete Mengen cis-3-Pentennitril, trans-2-Pentennitril, cis-2-Pentennitril, 4-Pentennitril und geringe Mengen (Z)-2-Methyl-2-butennitril und (E)-2-Methyl-2-butennitril, sowie die Katalysatorbestandteile und Katalysatorabbauprodukte.

Strom 1 wird in einem Verfahrensschritt (b) in einer Destillationskolonne K1 zugeführt, die mit einem Fallfilmverdampfer ausgestattet ist und als Abtriebskolonne mit Kolonneneinbauten betrieben wird, die 8 destillative Trennstufen zur Verfügung stellen. Die Destillationskolonne K1 wird am Kopf mit einem Kondensator betrieben, der mit kondensierten Material aus dem Rücklaufbehälter gespült wird. Die Destillationskolonne K1 wird bei einem absoluten Druck von 1,3 bar Kopfdruck, 278 K Kondensationstemperatur und 403 K Sumpfabzugstemperatur betrieben.

In die Destillationskolonne K1 wird der Nitrilrückführstrom 7 aus Kolonne K3, wie weiter unten beschrieben, zurückgeführt.

In den Kondensatsammelbehälter der Destillationskolonne K1 werden 34 kg/h handelsübliches 1,3-Butadien dosiert, enthaltend 0,25 Gew.-% cis-2-Buten, das durch Kontakt mit Aluminiumoxid behandelt wurde, wobei der Wassergehalt des eingesetzten 1,3-Butadien auf weniger als 10 Gew.-ppm H₂O und der Gehalt an Stabilisator tert.-Butylbrenzkatechin auf weniger als 10 ppm reduziert wurde.

Aus dem Kondensatsammelbehälter der Verdampferstufe wird Strom 2 als Summe von rückgeführtem und frisch zudosiertem 1,3-Butadien abgezogen und zum Reaktor R1a, wie vorher beschrieben, zurückgeführt.

Über Sumpf der Destillationskolonne K1 werden 76 kg/h eines Stromes 3 erhalten, der 0,8 Gew.-% 1,3-Butadien, 12 Gew.-% 2-Methyl-3-butennitril, 69 Gew.-% trans-3-Pentennitril und weitere Pentennitrilisomere sowie zusätzlich die Katalysatorbestandteile enthält. Die Zusammensetzung des Sumpfaustrags der Verdampferstufe entspricht einem Umsatzgrad von 75 Gew.-% 2-Methyl-3-butennitril zu trans-3-Pentennitril im Sumpf der Verdampferstufe K1.

Strom 3 wird in einem Verfahrensschritt (c) in eine Verdampferstufe B2 gefahren, die mit Fallfilmverdampfer und Kondensator ausgestattet ist und bei einem absoluten Druck von 220 mbar und 381 K Sumpfabzugstemperatur betrieben wird.

Aus der Verdampferstufe B2 wird ein Strom 5 gasförmig gewonnen (58 kg/h), enthaltend 97 Gew.-% Pentennitrilisomere sowie ca. 1 Gew.-% 1,3-Butadien und in geringerem Umfang (E)-2-Methyl-2-butennitril, (Z)-2-Methyl-2-butennitril und Vinylcyclohexen.

An der Verdampferstufe B2 wird über Sumpf der Katalysator-Strom 6 erhalten (17 kg/h), enthaltend 0,9 Gew.-% Ni(0). 0,3 Gew.-% 2-Methyl-3-butennitril und 42 Gew.-% restliche Pentennitrile. Der Strom 6 wird größtenteils (Strom 6a) in den Reaktor R1 zurückgefahren (10 kg/h). Der Rest (Strom 6b) wird einer Regenerierung (REG), beispielsweise gemäß US 2003/0100442 zugeführt und kann nach der Regenerierung in einer Hydrocyanierung von 3-Pentennitril verwendet werden oder in das erfindungsgemäße Verfahren in den Verfahrensschritt zur Hydrocyanierung von 1,3-Butadien zurückgefahren werden.

Der Strom 5 wird kondensiert und flüssig in einem Verfahrensschritt (d) zu einer Destillationskolonne K3 gefahren, die mit Zwangsumlaufverdampfer und Kopfkondensator sowie mit strukturierter Packung ausgestattet ist, die 50 theoretische Trennstufen erzeugen. Die Kolonne K3 wird bei einem absoluten Druck von 0,200 bar Kopfdruck, 342 K Kopftemperatur und 366 K Sumpfabzugstemperatur betrieben.

Am Kopf der Kolonnen K3 wird ein Strom 10 erhalten, enthaltend 10 Gew.-% 1,3-Butadien, 18 Gew.-% (Z)-2-Methyl-2-butennitril, 68 Gew.-% 2-Methyl-3-butennitril sowie weitere Pentennitril-Isomere und Vinylcyclohexen. Das Rücklaufverhältnis der Kolonne K3 wird so eingestellt, dass im Kopfabzugsstrom 18 Gew.-% (Z)-2-Methyl-2-butennitril enthält.

An einem flüssigen Seitenabzug der Kolonne K3 werden 8 kg/h eines Strom 7 erhalten, enthaltend 0,5 Gew.-% trans-3-Pentennitril, 85 Gew.-% 2-Methyl-3-butennitril, 5 Gew.-% (Z)-2-Methyl-2-butennitril und 10 Gew.-% Vinylcyclohexen. Strom 7 wird in die Destillationskolonne K1 in Schritt (b) zurückgefahren.

Über Sumpf der Kolonne K3 werden 47 kg/h des Stromes 8 erhalten, enthaltend in Summe 98 Gew.-% trans-3-Pentennitril, cis-3-Pentennitril und 4-Pentennitril sowie 100 ppm 2-Methyl-3-butennitril und ca. 1 Gew.-% (E)-2-Methyl-2-butennitril.

In Beispiel 5 wird die Destillationsvorrichtung K1 aus Beispiel 1 als Destillationskolonne mit Abtriebsteil ausgeführt, die Destillationsvorrichtung K2 aus Beispiel 1 kann hier als einstufige Verdampfung B2 ausgeführt werden, da der 2-Methyl-3-butennitril-Gehalt im Zulauf zu B2 im Vergleich zu Beispielen 1, 2 oder 3 durch vorherige Isomerisierung deutlich erniedrigt ist. Im Vergleich zu Beispiel 4 führt die Verfahrensweise nach Beispiel 5 zu geringeren Butadien-Verlusten, aber den Katalysatorstrom wird nach wie vor stärker als in Beispiel 1 oder 2 belastet.

## Patentansprüche

1. Verfahren zur Herstellung von 3-Pentennitril, **gekennzeichnet durch** die folgenden Verfahrensschritte:
(a) Umsetzung von 1,3-Butadien mit Cyanwasserstoff an mindestens einem Katalysator unter Erhalt eines Stromes 1, der 3-Pentennitril, 2-Methyl-3-butennitril, den mindestens einen homogen gelösten Nickel(0)-Katalysator, der mit Phosphorliganden stabilisiert ist und die Phosphorliganden ausgewählt sind aus der Gruppe, bestehend aus Phosphinen, Phosphiten, Phosphiniten und Phosphoniten und 1,3-Butadien enthält,
(b) Destillation des Stromes 1 in einer Kolonne unter Erhalt eines an 1,3-Butadien reichen Stromes 2 als Kopfprodukt und eines an 1,3-Butadien armen Stromes 3 als Sumpfprodukt, der 3-Pentennitril, den mindestens einen Katalysator und 2-Methyl-3-butennitril enthält,
(c) Destillation des Stromes 3 in einer Kolonne unter Erhalt eines Stromes 4 als Kopfprodukt, der 1,3-Butadien enthält, eines Stromes 5 an einem Seitenabzug der Kolonne, der 3-Pentennitril und 2-Methyl-3-butennitril enthält, und eines Stromes 6 als Sumpfprodukt, der den mindestens einen Katalysator enthält,
(d) Destillation des Stromes 5 unter Erhalt eines Stromes 7 als Kopfprodukt, der 2-Methyl-3-butennitril enthält, und eines Stromes 8 als Sumpfprodukt, der 3-Pentenntril enthält,
mit der Maßgabe, dass in den Verfahrensschritten (b) und (c) die Sumpftemperaturen 140°C nicht übersteigen und die Summe der mittleren Verweilzeiten in den Destillationsvorrichtungen in den Verfahrensschritten (b) und (c) zusammen nicht größer als 10 Stunden ist.

2. Verfahren nach Anspruch 1, **dadurch gekennzeichnet, dass** der an 1,3-Butadien reiche Strom 2 aus Verfahrensschritt (b) zumindest teilweise in den Verfahrensschritt (a) zurückgeführt wird.

3. Verfahren nach Anspruch 1 oder 2, **dadurch gekennzeichnet, dass** in Verfahrensschritt (c) über Sumpf der Strom 6 mit einer Konzentration von 2-Methyl-3-butennitril gewonnen wird, die im Vergleich zum Strom 5 erniedrigt ist, wobei sich die Erniedrigung auf das Verhältnis der Konzentrationen von 2-Methyl-3-butennitril zu trans-3-Pentenntril bezieht.

4. Verfahren nach einem der Ansprüche 1 bis 3, **dadurch gekennzeichnet, dass** der Strom 6 aus Verfahrensschritt (c) zumindest teilweise in den Verfahrensschritt (a) zurückgeführt wird.

5. Verfahren nach einem der Ansprüche 1 bis 4, **dadurch gekennzeichnet, dass** der Strom 4 aus Verfahrensschritt (c) zumindest teilweise in Verfahrensschritt (a) und/oder (b) zurückgeführt wird.

6. Verfahren nach einem der Ansprüche 1 bis 5, **dadurch gekennzeichnet, dass** der Strom 5 am Seitenabzug in Verfahrensschritt (c) dampfförmig entnommen wird.

7. Verfahren nach einem der Ansprüche 1 bis 6, **dadurch gekennzeichnet, dass** der Strom 7 aus Verfahrensschritt (d) zumindest teilweise in Verfahrensschritt (a) und/oder Verfahrensschritt (b) zurückgeführt wird.

8. Verfahren nach einem der Ansprüche 1 bis 7, **dadurch gekennzeichnet, dass** in Verfahrensschritt (c) 1 bis 50 destillative Trennstufen zwischen der Position des Seitenabzugs und dem Sumpf sind.

9. Verfahren nach einem der Ansprüche 1 bis 8, **dadurch gekennzeichnet, dass** der Anteil an 2-Methyl-3-butennitril in dem in Verfahrensschritt (c) erhaltenen Katalysatorstrom 6 0 bis 5 Gew.-% beträgt.

## Claims

1. A process for preparing 3-pentenenitrile, **characterized by** the following process steps:
(a) reacting 1,3-butadiene with hydrogen cyanide over at least one catalyst to obtain a stream 1 which comprises 3-pentenenitrile, 2-methyl-3-butenenitrile, the at least one homogeneously dissolved nickel(0) catalyst which is stabilized with phosphorus ligands and the phosphorus ligands are selected from the group consisting of phosphines, phosphites, phosphinites and phosphonites, and 1,3-butadiene,
(b) distilling stream 1 in a column to obtain a high-1,3-butadiene stream 2 as the top product and a low-1,3-butadiene stream 3 as the bottom product which comprises 3-pentenenitrile, the at least one catalyst and 2-methyl-3-butenenitrile,
(c) distilling stream 3 in a column to obtain a stream 4 as the top product which comprises 1,3-butadiene, a stream 5 which comprises 3-pentenenitrile and 2-methyl-3-butenenitrile at a side draw of the column, and a stream 6 as the bottom product which comprises the at least one catalyst,
(d) distilling stream 5 to obtain a stream 7 as the top product which comprises 2-methyl-3-butenenitrile, and a stream 8 as the bottom product which comprises 3-pentenenitrile,
with the proviso that, in process steps (b) and (c), the bottom temperatures do not exceed 140°C and the sum of the average residence times in the distillation apparatus in process steps (b) and (c) together is not more than 10 hours.

2. The process according to claim 1, wherein the high-1,3-butadiene stream 2 from process step (b) is recycled at least partly into process step (a).

3. The process according to claim 1 or 2, wherein, in process step (c), stream 6 is obtained via the bottom with a concentration of 2-methyl-3-butenenitrile which is lowered in comparison to stream 5, the lowering being based on the ratio of the concentrations of 2-methyl-3-butenenitrile to trans-3-pentenenitrile.

4. The process according to any of claims 1 to 3, wherein stream 6 from process step (c) is recycled at least partly into process step (a).

5. The process according to any of claims 1 to 4, wherein stream 4 from process step (c) is recycled at least partly into process step (a) and/or (b).

6. The process according to any of claims 1 to 5, wherein stream 5 is withdrawn in vaporous form at the side draw in process step (c).

7. The process according to any of claims 1 to 6, wherein stream 7 from process step (d) is recycled at least partly into process step (a) and/or process step (b).

8. The process according to any of claims 1 to 7, wherein, in process step (c), there are from 1 to 50 distillative separation stages between the position of the side draw and the column bottom.

9. The process according to any of claims 1 to 8, wherein the proportion of 2-methyl-3-butenenitrile in the catalyst stream 6 obtained in process step (c) is from 0 to 5% by weight.

## Revendications

1. Procédé de fabrication de 3-pentène-nitrile, **caractérisé par** les étapes de procédé suivantes :
(a) la réaction de 1,3-butadiène avec du cyanure d'hydrogène sur au moins un catalyseur pour obtenir un courant 1, qui contient du 3-pentène-nitrile, du 2-méthyl-3-butène-nitrile, le ou les catalyseurs de nickel (0) homogènes dissous, qui sont stabilisés avec des ligands phosphore, les ligands phosphore étant choisis dans le groupe constitué par les phosphines, les phosphites, les phosphinites et les phosphonites, et du 1,3-butadiène,
(b) la distillation du courant 1 dans une colonne pour obtenir un courant 2 riche en 1,3-butadiène en tant que produit de tête et un courant 3 pauvre en 1,3-butadiène en tant que produit de fond, qui contient du 3-pentène-nitrile, le ou les catalyseurs et du 2-méthyl-3-butène-nitrile,
(c) la distillation du courant 3 dans une colonne pour obtenir un courant 4 en tant que produit de tête, qui contient du 1,3-butadiène, un courant 5 par une sortie latérale de la colonne, qui contient du 3-pentène-nitrile et du 2-méthyl-3-butène-nitrile, et un courant 6 en tant que produit de fond, qui contient le ou les catalyseurs,
(d) la distillation du courant 5 pour obtenir un courant 7 en tant que produit de tête, qui contient du 2-méthyl-3-butène-nitrile, et un courant 8 en tant que produit de fond, qui contient du 3-pentène-nitrile,
à condition que les températures de fond lors des étapes de procédé (b) et (c) ne dépassent pas 140 °C et que la somme des temps de séjour moyens dans les dispositifs de distillation des étapes de procédé (b) et (c) ne soit pas supérieure à 10 heures.

2. Procédé selon la revendication 1, **caractérisé en ce que** le courant 2 riche en 1,3-butadiène de l'étape de procédé (b) est recyclé au moins en partie dans l'étape de procédé (a).

3. Procédé selon la revendication 1 ou 2, **caractérisé en ce que** le courant 6 est obtenu par le fond lors de l'étape de procédé (c) avec une concentration en 2-méthyl-3-butène-nitrile qui est réduite en comparaison du courant 5, la réduction se rapportant au rapport entre les concentrations de 2-méthyl-3-butène-nitrile et de trans-3-pentène-nitrile.

4. Procédé selon l'une quelconque des revendications 1 à 3, **caractérisé en ce que** le courant 6 de l'étape de procédé (c) est recyclé au moins en partie dans l'étape de procédé (a).

5. Procédé selon l'une quelconque des revendications 1 à 4, **caractérisé en ce que** le courant 4 de l'étape de procédé (c) est recyclé au moins en partie dans l'étape de procédé (a) et/ou (b).

6. Procédé selon l'une quelconque des revendications 1 à 5, **caractérisé en ce que** le courant 5 est soutiré sous forme gazeuse par la sortie latérale lors de l'étape de procédé (c).

7. Procédé selon l'une quelconque des revendications 1 à 6, **caractérisé en ce que** le courant 7 de l'étape de procédé (d) est recyclé au moins en partie dans l'étape de procédé (a) et/ou l'étape de procédé (b).

8. Procédé selon l'une quelconque des revendications 1 à 7, **caractérisé en ce qu'**il y a 1 à 50 étapes de séparation par distillation entre la position de la sortie latérale et du fond à l'étape de procédé (c).

9. Procédé selon l'une quelconque des revendications 1 à 8, **caractérisé en ce que** la proportion de 2-méthyl-3-butène-nitrile dans le courant de catalyseur obtenu lors de l'étape de procédé (c) est de 60 à 5 % en poids.
